# EUROPÄISCHE PATENTSCHRIFT

(11) **EP 2 176 316 B1**
(45) Veröffentlichungstag und Bekanntmachung des Hinweises auf die Patenterteilung: **23.03.2011**
(21) Anmeldenummer: 08785168.9
(22) Anmeldetag: 29.07.2008
(51) Int. Cl.: C08G 65/327, C08L 71/02

(54) **PHOSPHORSÄUREESTER ENTHALTEND ÜBER POLYOL-EINHEITEN VERBRÜCKTE PHOSPHORATOME**
PHOSPHORIC ACID ESTERS CONTAINING PHOSPHORUS ATOMS BRIDGED BY POLYOL UNITS
ESTERS D'ACIDE PHOSPHORIQUE CONTENANT DES ATOMES DE PHOSPHORE PONTÉS PAR DES MOTIFS POLYOL

(30) Priorität: 02.08.2007 DE 102007036186
(43) Veröffentlichungstag der Anmeldung: 21.04.2010
(73) Patentinhaber: Clariant Finance (BVI) Limited, Road Town, Tortola (VG)
(72) Erfinder: KLUG, Peter, 63762 Grossostheim (DE); SCHERL, Franz-Xaver, 84508 Burgkirchen (DE); SIMSCH, Waltraud, 65779 Kelkheim (DE); OBERHAUSER, Adelgunde, 84524 Neuötting (DE)
(74) Vertreter: Paczkowski, Marcus
(86) Internationale Anmeldenummer: PCT/EP2008/006220
(87) Internationale Veröffentlichungsnummer: WO 2009/015858

(56) Entgegenhaltungen:
- US-A- 3 275 667
- US-A- 4 180 532

## Beschreibung

Die Erfindung betrifft Ester aus Phosphorsäure oder Phosphorsäurederivaten, Fettalkohole, der gegebenenfalls alkoxyliert ist, und Polyol, sowie deren Verwendung als Assoziativverdicker, insbesondere in kosmetischen, pharmazeutischen oder dermatologischen Zusammensetzungen.

An kosmetische Produkte werden hohe Anforderungen gestellt. Sie sollen ein klares Erscheinungsbild zeigen, toxikologisch und ökotoxikologisch unbedenklich sein, ein angenehmes Hautgefühl erzeugen und ein ausgezeichnetes rheologisches Verhalten haben, das über einen breiten pH-Bereich konstant ist.

Wasser- oder lösungsmittelhaltige Mehrkomponentensysteme wie Emulsionen oder Suspensionen werden häufig aus ökonomischen Gründen, aus anwendungstechnischen Gründen oder aus Stabilitätsgründen auf höhere Viskositäten eingestellt bzw. verdickt.

So kann z. B. durch Erhöhung der Viskosität der externen oder internen Phase von Emulsionen oder Suspensionen erreicht werden, dass die Zeit bis zur Entmischung der Komponenten eines solchen Systems deutlich verlängert werden kann, was sich in einer Verlängerung der Lagerzeit bemerkbar macht. Durch Erhöhung der Viskosität wird auch bei vielen Produkten deren gleichmäßige Verteilbarkeit insbesondere auf unebenen Flächen verbessert.

Durch die gleichmäßigere Verteilung und verlängerte Einwirkdauer wird so die Wirksamkeit erhöht. Neben den erwähnten anwendungstechnischen Vorteilen bietet die hohe Viskosität solcher Präparate auch weitere Vorteile bei der Herstellung, Verpackung, Abfüllung und Lagerung sowie beim Transport.

In der Fachliteratur wird eine Vielzahl von unterschiedlichen Systemen angegeben, um die rheologischen Eigenschaften von wässrigen oder lösungsmittelhaltigen Systemen, Emulsionen oder Suspensionen einzustellen. Bekannt sind beispielsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z. B. Polyvinylalkohole, Polyacrylamide, Polyacrylsäure und verschiedene Salze der Polyacrylsäure, Polyvinylpyrrolidon, Polyvinylmethylether, Polyethylenoxide, Copolymere aus Maleinsäureanhydrid und Vinylmethylether, sowie diverse Mischungen und Copolymerisate aus den oben angegebenen Verbindungen.

Die genannten Verbindungen zeigen jedoch bei der Anwendung vielfältige Nachteile. So sind z. B. die Cellulosederivate bzw. allgemein die auf natürlichen Rohstoffen basierenden Materialien und die daraus resultierenden Formulierungen sehr anfällig gegen Bakterien. Anwendungstechnisch fallen sie zumeist durch die Bildung unangenehmer, "faden ziehender" Gele auf. Fettsäurepolyethylenglykolester neigen in Gegenwart von Wasser zur Hydrolyse, die dabei entstehenden unlöslichen Fettsäuren verursachen unerwünschte Trübungen. Verdickungsmittel natürlichen Ursprungs (z. B. Agar-Agar oder Traganth) weisen je nach Herkunft eine stark schwankende Zusammensetzung auf.

US 5,129,462 beschreibt Shampoo-Formulierungen, enthaltend Polyethylenglykol-Polyolfettsäureester, insbesondere Polyethylenglykol Pentaerythritolfettsäureester als Verdickungsmittel. Die Verarbeitung und Formulierbarkeit dieser Verbindungsklasse ist durch deren hohe Schmelzpunkte bzw. Stockpunkte beeinträchtigt.

EP 1 518 900 und EP 1 344 518 offenbaren kosmetische und pharmazeutische Zubereitungen enthaltend oxalkylierte Polyglycerinester als Verdicker, Dispergiermittel für wässrige, wässrig-alkoholische und wässrig-tensidische Zubereitungen und als Emulgatoren, Suspendiermittel mit verdickender Wirkung und Konsistenzgeber für Emulsionen und Suspensionen.

Die in US 5,129,462, EP 1 518 900 und EP 1 344 518 beschriebenen Assoziativverdicker haben einerseits noch Verbesserungspotential in Bezug auf ihre Verdickungsleistung, speziell in rein wässrigen Systemen, wo sie nur trübe Gele bilden, aber auch in Bezug auf ihre Stabilität bei niedrigem pH-Wert. Bei pH-Werten unterhalb 5 sind ihre Gele und verdickte Tensidlösungen nicht lagerstabil, sondern verlieren sehr schnell an Viskosität.

Aufgabe der Erfindung war es daher, eine neuartige Substanzklasse zur Verfügung zu stellen, die für den Einsatz in kosmetischen Produkten geeignet ist, in den Formulierungen ein klares Erscheinungsbild ergibt und auch in einem stark sauren Medium ein hohes Verdickungsvermögen bei Temperaturbelastung und langen Lagerzeiten bewirkt und diese Eigenschaften mit exzellenter Verdickungsleistung kombiniert.

Es wurde überraschend gefunden, dass diese Aufgabe gelöst wird durch Ester der Phosphorsäure oder Ester von Phosphorsäurederivaten mit gegebenenfalls alkoxylierten Fettakoholen, die dadurch gekennzeichnet sind, dass mindestens 2 Phosphoratome über Gruppen, die sich von Polyolen mit mehr als 2 OH-Gruppen oder den entsprechenden alkoxylierten Polyolen ableiten, verbrückt sind.

Gegenstand der vorliegenden Erfindung sind daher Phosphorsäureester enthaltend
A) eine oder mehrere Struktureinheiten abgeleitet von Substanzen der Komponente a), wobei die Substanzen der Komponente a) ausgewählt sind aus Orthophosphorsäure und einem oder mehreren ihrer Derivate, und wobei das eine oder die mehreren Derivate der Orthophosphorsäure vorzugsweise ausgewählt sind aus Polyphosphorsäure, Tetraphosphordecaoxid, Phosphoroxychlorid und Phosphorpentachlorid,
B) eine oder mehrere Struktureinheiten abgeleitet von Substanzen der Komponente b), wobei die Substanzen der Komponente b) ausgewählt sind aus einer oder mehreren Verbindungen der Formel (I)

   R²-O-(CH₂CH₂O)ᵤ(C₃H₆O,(DO)_{w}-H (I)

   worin
   R² für eine lineare oder verzweigte, gesättigte Alkylgruppe mit 6 bis 30, vorzugsweise 8 bis 22 und besonders bevorzugt 12 bis 18 Kohlenstoffatomen, oder für eine lineare oder verzweigte, ein- oder mehrfach ungesättigte Alkenylgruppe mit 6 bis 30, vorzugsweise 8 bis 22 und besonders bevorzugt 12 bis 18 Kohlenstoffatomen, steht,
   D für eine lineare oder verzweigte gesättigte Alkylengruppe mit 4 bis 20 Kohlenstoffatomen, für eine lineare oder verzweigte ein- oder mehrfach ungesättigte Alkenylengruppe mit 4 bis 20 Kohlenstoffatomen oder für -CH(Phenyl)CH₂- steht,
   u für eine Zahl von 0 bis 200, vorzugsweise von 2 bis 150, besonders bevorzugt von 5 bis 100, insbesondere bevorzugt von 10 bis 50, steht,
   v für eine Zahl von 0 bis 100, vorzugsweise von 0 bis 50, besonders bevorzugt von 0 bis 20, steht,
   w für eine Zahl von 0 bis 100, vorzugsweise von 0 bis 50, besonders bevorzugt von 0 bis 20, steht, und
   wobei die Gruppen CH₂CH₂O, C₃H₆O und DO aus den Verbindungen der Formel (I) blockartig oder statistisch verteilt angeordnet sein können, und
C) eine oder mehrere Struktureinheiten abgeleitet von Substanzen der Komponente c), wobei die Substanzen der Komponente c) ausgewählt sind aus einem oder mehreren Polyolen mit mehr als 2 OH-Gruppen, welche auch eine oder mehrere Alkoxylatgruppen tragen können und wobei die Alkoxylatgruppen jeweils aufgebaut sind aus einer oder mehreren Einheiten ausgewählt aus CH₂CH₂O-, C₃H₆O- und C₄H₈O-Einheiten, die innerhalb der Alkoxylatgruppen jeweils blockartig oder statistisch verteilt angeordnet sein können,
und wobei die Phosphorsäureester mindestens 2 über eine Struktureinheit abgeleitet von den Polyolen mit mehr als 2 OH-Gruppen oder abgeleitet von den Polyolen mit mehr als 2 OH-Gruppen, die ein oder mehrere der Alkoxylatgruppen tragen, verbrückte Phosphoratome pro Molekül enthalten.

Die erfindungsgemäßen Phosphorsäureester enthalten keine Sauerstoff-Sauerstoff-Bindung -O-O-. Die Struktureinheiten abgeleitet von den Substanzen der Komponenten a), b) und c) sind über nur ein Sauerstoffatom -O- aneinander gebunden.

In einer bevorzugten Ausführungsform der Erfindung sind die erfindungsgemäßen Phosphorsäureester dadurch gekennzeichnet, dass die Substanzen der Komponente c) ausgewählt sind aus Glycerin, Diglycerin, Polyglycerin, Pentaerythrit, Dipentaerythrit, Pentaerythritoligomeren, Trimethylolpropan, Threit, Erythrit, Adonit, Arabit, Xylit, Mannit, Sorbitol, Inosit, Glucose, Mannose, Fructose, Sorbose, Arabinose, Xylose, Ribose, Mannopyranose, Galactopyranose, Glucopyranose, Maltose, Saccharose, Aminozucker, Ascorbinsäure, Glucamiden und Gluconamiden, welche auch eine oder mehrere Alkoxylatgruppen tragen können und wobei die Alkoxylatgruppen jeweils aufgebaut sind aus einer oder mehreren Einheiten ausgewählt aus CH₂CH₂O-, C₃H₆O- und C₄H₈O-Einheiten, die innerhalb der Alkoxylatgruppen jeweils blockartig oder statistisch verteilt angeordnet sein können.

In einer besonders bevorzugten Ausführungsform der Erfindung sind die erfindungsgemäßen Phosphorsäureester dadurch gekennzeichnet, dass die Substanzen der Komponente c) ausgewählt sind aus Pentaerythrit, Glycerin und Diglycerin, vorzugsweise Pentaerythrit, welche auch eine oder mehrere Alkoxylatgruppen tragen können und wobei die Alkoxylatgruppen jeweils aufgebaut sind aus einer oder mehreren Einheiten ausgewählt aus CH₂CH₂O-, C₃H₆O- und C₄H₈O-Einheiten, die innerhalb der Alkoxylatgruppen jeweils blockartig oder statistisch verteilt angeordnet sein können.

In einer weiteren bevorzugten Ausführungsform der Erfindung sind die erfindungsgemäßen Phosphorsäureester dadurch gekennzeichnet, dass die Substanzen der Komponente c) eine oder mehrere Alkoxylatgruppen tragen. Unter diesen erfindungsgemäßen Phosphorsäureestern sind diejenigen bevorzugt, worin die Alkoxylatgruppen der Substanzen der Komponente c) aus CH₂CH₂O-Gruppen bestehen und die Anzahl der CH₂CH₂O-Gruppen pro Polyol-Molekül mit mehr als 2 OH-Gruppen von 1 bis 150, bevorzugt von 5 bis 130 und besonders bevorzugt von 10 bis 110 beträgt.

In einer weiteren bevorzugten Ausführungsform der Erfindung sind die erfindungsgemäßen Phosphorsäureester dadurch gekennzeichnet, dass die Substanzen der Komponente b) ausgewählt sind aus einer oder mehreren Verbindungen der Formel (II),

R-O-(CH₂CH₂O)ᵤ₁ (C₃H₆O)ᵥ₁-H (II)

worin
R² für eine lineare oder verzweigte, gesättigte Alkylgruppe mit 6 bis 30, vorzugsweise 8 bis 22 und besonders bevorzugt 12 bis 18 Kohlenstoffatomen, oder für eine lineare oder verzweigte, ein- oder mehrfach ungesättigte Alkenylgruppe mit 6 bis 30, vorzugsweise 8 bis 22 und besonders bevorzugt 12 bis 18 Kohlenstoffatomen, steht,
u1 für eine Zahl von 1 bis 200, vorzugsweise von 2 bis 150, besonders bevorzugt von 5 bis 100 und insbesondere bevorzugt von 10 bis 50, steht, und
v1 für eine Zahl von 1 bis 100, vorzugsweise von 1 bis 50 und besonders bevorzugt von 1 bis 20, steht,
und wobei die CH₂CH₂O- und C₃H₆O- Einheiten blockartig oder statistisch verteilt angeordnet sein können.

In einer weiteren bevorzugten Ausführungsform der Erfindung sind die erfindungsgemäßen Phosphorsäureester dadurch gekennzeichnet, dass die Substanzen der Komponente b) ausgewählt sind aus einer oder mehreren Verbindungen der Formel (III),

R²-O-(CH₂CH₂O)ᵤ₁-H (III)

worin
R² für eine lineare oder verzweigte, gesättigte Alkylgruppe mit 6 bis 30, vorzugsweise 8 bis 22 und besonders bevorzugt 12 bis 18 Kohlenstoffatomen, oder für eine lineare oder verzweigte, ein- oder mehrfach ungesättigte Alkenylgruppe mit 6 bis 30, vorzugsweise 8 bis 22 und besonders bevorzugt 12 bis 18 Kohlenstoffatomen, steht, und
u1 für eine Zahl von 1 bis 200, vorzugsweise von 2 bis 150, besonders bevorzugt von 5 bis 100 und insbesondere bevorzugt von 10 bis 50, steht.

In einer weiteren bevorzugten Ausführungsform der Erfindung sind die erfindungsgemäßen Phosphorsäureester dadurch gekennzeichnet, dass die eine oder die mehreren Struktureinheiten abgeleitet von der einen oder den mehreren Verbindungen der Formel (I) solche Struktureinheiten sind, worin u für eine Zahl von 1 bis 200, vorzugsweise von 2 bis 150, besonders bevorzugt von 5 bis 100 und insbesondere bevorzugt von 10 bis 50, steht, v und w 0 sind, und der Rest R²-O- abgeleitet ist von Alkoholen ausgewählt aus Octanol, Decanol, Dodecanol, Tetradecanol, Hexadecanol, Octadecanol, Eicosanol, Behenylalkohol, Fettalkoholen mit C-Ketten-Schnitten zwischen 8 und 22, vorzugsweise C₁₀/C₁₂-Fettalkohol, C₁₂/C₁₄-Fettalkohol, C₁₂/C₁₅- Fettalkohol und C₁₆/C₁₈-Fettalkohol, verzweigten Fettalkoholen, vorzugsweise Guerbetalkoholen, und einfach ungesättigten Fettalkoholen, vorzugsweise Delta-9-cis-Hexadecanol, Delta-9-cis-Octadecanol, trans-9-Octadecanol und cis-Delta-11-Octadecanol.

In einer besonders bevorzugten Ausführungsform der Erfindung sind die erfindungsgemäßen Phosphorsäureester dadurch gekennzeichnet, dass die eine oder die mehreren Struktureinheiten abgeleitet von der einen oder den mehreren Verbindungen der Formel (I) Struktureinheiten sind abgeleitet von C_{16/18}-Fettalkoholethoxylaten mit 10-50 Ethylenoxid-Einheiten, vorzugsweise abgeleitet von Substanzen ausgewählt aus C_{16/18}-Fettalkoholethoxylat mit 11 Ethylenoxid-Einheiten, C_{16/18}-8-Fettalkoholethoxylat mit 25 Ethylenoxid-Einheiten und C_{16/18}-Fettalkoholethoxylat mit 50 Ethylenoxid-Einheiten.

In einer weiteren bevorzugten Ausführungsform der Erfindung die erfindungsgemäßen Phosphorsäureester dadurch gekennzeichnet, dass die Gesamtzahl der in den Phosphorsäureestern enthaltenen Ethylenoxideinheiten in den Struktureinheiten abgeleitet von den Substanzen der Komponente b) und den Substanzen der Komponente c) zusammen pro Fettalkoholendgruppe hervorgegangen aus den Verbindungen der Formel (I) von 30 bis 100 und vorzugsweise von 40 bis 80 ist.

In einer weiteren besonders bevorzugten Ausführungsform der Erfindung sind die erfindungsgemäßen Phosphorsäureester dadurch gekennzeichnet, dass sie erhältlich sind aus der Umsetzung von
a) 5 bis 10 Mol eines C₁₂-C₂₂-Fettalkoholethoxylats, vorzugsweise eines C_{16/18}-Fettalkoholethoxylats, mit 10-50 Ethylenoxid-Einheiten und vorzugsweise mit 11 oder 25 Ethylenoxid-Einheiten,
b) 1 Mol eines Polyols ausgewählt aus Pentaerythrit, Glycerin und Diglycerin jeweils ethoxyliert mit 50 bis 150 Ethylenoxideinheiten und
c) 2 bis 5 Mol Orthophosphorsäure oder einem oder mehreren ihrer Derivate, wobei das eine oder die mehreren Derivate der Orthophosphorsäure vorzugsweise ausgewählt sind aus Polyphosphorsäure, Tetraphosphordecaoxid, Phosphoroxychlorid und Phosphorpentachlorid.

In einer insbesondere bevorzugten Ausführungsform der Erfindung sind die erfindungsgemäßen Phosphorsäureester dadurch gekennzeichnet, dass sie erhältlich sind aus der Umsetzung von
a) 6 bis 10 Mol, vorzugsweise 8 Mol, eines C₁₂-C₂₂-Fettalkoholethoxylats, vorzugsweise eines C_{16/18}- Fettalkoholethoxylats, mit 10-50 Ethylenoxid-Einheiten, vorzugsweise mit 11 oder 25 Ethylenoxid-Einheiten,
b) 1 Mol Pentaerythrit, ethoxyliert mit 50 bis 150, vorzugsweise 100, Ethylenoxideinheiten, und
c) 3 bis 5 Mol, vorzugsweise 4 Mol, Orthophosphorsäure oder einem oder mehreren ihrer Derivate, wobei das eine oder die mehreren Derivate der Orthophosphorsäure vorzugsweise ausgewählt sind aus Polyphosphorsäure, Tetraphosphordecaoxid, Phosphoroxychlorid und Phosphorpentachlorid.

Unter den soeben genannten erfindungsgemäßen Phosphorsäureestern sind wiederum diejenigen insbesondere bevorzugt, die erhältlich sind aus der Umsetzung von
a) 8 Mol eines C₁₂-C₂₂-Fettalkoholethoxylats, vorzugsweise eines C_{16/18}-Fettalkoholethoxylats, mit 10-50 Ethylenoxid-Einheiten und vorzugsweise mit 11 oder 25 Ethylenoxid-Einheiten,
b) 1 Mol Pentaerythrit, ethoxyliert mit 100 Ethylenoxideinheiten und
c) 4 Mol Orthophosphorsäure.

In einer weiteren insbesondere bevorzugten Ausführungsform der Erfindung sind die erfindungsgemäßen Phosphorsäureester dadurch gekennzeichnet, dass sie erhältlich sind aus der Umsetzung von
a) 6 Mol eines C₁₂-C₂₂-Fettalkoholethoxylats, vorzugsweise eines C_{16/18}-Fettalkoholethoxylats, mit 10-50 Ethylenoxid-Einheiten und vorzugsweise mit 11 oder 25 Ethylenoxid-Einheiten,
b) 1 Mol Glycerin, ethoxyliert mit 100 Ethylenoxideinheiten und
c) 3 Mol Orthophosphorsäure

In einer weiteren insbesondere bevorzugten Ausführungsform der Erfindung sind die erfindungsgemäßen Phosphorsäureester dadurch gekennzeichnet, dass sie erhältlich sind aus der Umsetzung von
a) 8 Mol eines C₁₂-C₂₂-Fettalkoholethoxylats, vorzugsweise eines C_{16/18}-Fettalkoholethoxylats, mit 10-50 Ethylenoxid-Einheiten und vorzugsweise mit 11 oder 25 Ethylenoxid-Einheiten,
b) 1 Mol Diglycerin, ethoxyliert mit 100 Ethylenoxideinheiten und
c) 4 Mol Orthophosphorsäure

In einer weiteren bevorzugten Ausführungsform der Erfindung sind die erfindungsgemäßen Phosphorsäüreester dadurch gekennzeichnet, dass mindestens 75 %, vorzugsweise von 80 bis 100 %, besonders bevorzugt von 85 bis 100 % der aus den Substanzen der Komponente a) theoretisch maximal erhältlichen veresterbaren Funktionen in den Phosphorsäureestern verestert.

Die übrigen freien Valenzen am Phosphoratom, d.h. die nicht veresterten der veresterbaren Funktionen, können Säuregruppen P-OH sein, aber auch Gruppen der Form P-OGegenion, wobei die Gegenionen ausgewählt sind aus Li⁺, Na⁺, K⁺, Mg⁺⁺, Ca⁺⁺, Al⁺⁺⁺, NH₄⁺ und quaternären Ammoniumionen [HNR^{a}R^{b}R^{c}]⁺, worin R^{a}, R^{b} und R^{c} unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine lineare Mono-Hydroxyalkylgruppe mit 2 bis 10 Kohlenstoffatomen, bevorzugt eine Mono-Hydroxyethyl- oder Mono-Hydroxypropylgruppe, sowie eine lineare oder verzweigte Di-Hydroxyalkylgruppe mit 3 bis 10 Kohlenstoffatomen, sein können.

Der Neutralisationsgrad der nicht veresterten Phosphorvalenzen (P-OH) in den erfindungsgemäßen Phosphörsäureestern kann zwischen 0 und 100 % betragen. In einer bevorzugten Ausführungsform der Erfindung ist der Neutralisationsgrad von 0 - 20 %. In einer anderen bevorzugten Ausführungsform der Erfindung ist der Neutralisationsgrad von 20,1 - 100 %.

In einer weiteren bevorzugten Ausführungsform der Erfindung sind die erfindungsgemäßen Phosphorsäureester dadurch gekennzeichnet, dass sie neben den Struktureinheiten abgeleitet von Substanzen der Komponenten a), b) und c), zusätzlich
D) eine oder mehrere Struktureinheiten abgeleitet von Substanzen der Komponente d), wobei die Substanzen der Komponente d) ausgewählt sind aus einem oder mehreren Diolen der Formel (IV)

   HO-(CH₂CH₂O)ₐ(C₃H₆O)_{b}(DO)_{c}-H (IV)
worin
D die Bedeutung wie in Formel (I) hat,
a für eine Zahl von 0 bis 800, vorzugsweise von 0 bis 250, besonders bevorzugt von 10 bis 200 und insbesondere bevorzugt von 20 bis 100, steht,
b für eine Zahl von 0 bis 100, vorzugsweise von 0 bis 50, steht,
c für eine Zahl von 0 bis 100, vorzugsweise von 0 bis 50, steht,
wobei die Summe a + b + c ≥ 1, vorzugsweise von 25 bis 250, ist und die Gruppen CH₂CH₂O, C₃H₆O und DO aus den Verbindungen der Formel (II) blockartig oder statistisch verteilt angeordnet sein können, enthalten.

Unter den soeben genannten erfindungsgemäßen Phosphorsäureestern sind wiederum diejenigen bevorzugt, die Struktureinheiten abgeleitet von Substanzen der Komponente d) enthalten, wobei die Substanzen der Komponente d) ausgewählt sind aus Ethylenglykol, Diethylenglykol, Triethylenglykol, Tetraethylenglykol, Polyethylenglykol (PEG) mit Molekulargewichten von 200 bis 35000, bevorzugt PEG 200, PEG 300, PEG 400, PEG 600, PEG 800, PEG 1000, PEG 1500, PEG 2000, PEG 3000, PEG 3350, PEG 4000, PEG 6000, PEG 8000, Propylenglykol, Dipropylenglykol, Tripropylenglykol, Polypropylenglykol, Polybutylenglykol, Copolymerisaten aus Ethylenoxid und Propylenoxid mit Molekulargewichten von 200 bis 35000, 1,2-Butandiol, 1,3-Butandiol, 1,4-Butandiol, 1,2-Pentandiol, 1,3-Pentandiol, 1,4-Pentandiol, 1,5-Pentandiol, 1,2-Hexandiol, 1,3-Hexandiol, 1,4-Hexandiol, 1,5-Hexandiol, 1,6-Hexandiol und 1,12-Dodecandiol.

Ein weiterer Gegenstand der vorliegenden Erfindung sind auch Mischungen enthaltend ein oder mehrere erfindungsgemäße Phosphorsäureester. In einer bevorzugten Ausführungsform der Erfindung können diese Mischungen auch Phosphorsäureester mit nur einem Phosphorsäureatom pro Molekül, insbesondere solche der Formel

[R²-O-(CH₂CH₂O)ᵤ(C₃H₆O)ᵥ(DO)_{w}-]₃P=O,

worin R², u, v, w und D die oben unter den Verbindungen der Formel (I) genannten Bedeutungen besitzen, enthalten. Unter diesen Mischungen sind wiederum diejenigen bevorzugt, die aus den genannten Phosphorsäureestern bestehen. Der Anteil der erfindungsgemäßen Phosphorsäureester in den erfindungsgemäßen Mischungen ist vorzugsweise größer als 50 Gew.-%, besonders bevorzugt von 70 bis 100 Gew.-% und insbesondere bevorzugt von 80 bis 100 Gew.-%. In einer anderen bevorzugten Ausführungsform der Erfindung bestehen die erfindungsgemäßen Mischungen aus den erfindungsgemäßen Phosphorsäureestern.

Weiterer Gegenstand der Erfindung ist auch ein Verfahren zur Herstellung der erfindungsgemäßen Phosphorsäureester.

Die Darstellung der erfindungsgemäßen Phosphorsäureester kann durch Umsetzung von Phosphorsäure oder deren Derivaten mit Alkohol, vorzugsweise Fettalkoholethoxylat, und Polyol mit mehr als 2 OH-Gruppen oder einem entsprechenden Alkoxylatgruppen-haltigen Polyol, sowie gegebenenfalls Diol, bei Temperaturen von 150 bis 250 °C, bevorzugt von 180 bis 240 °C und besonders bevorzugt von 200 bis 230 °C erfolgen, vorzugsweise ohne Zusatz eines Katalysators.

Ein weiterer Gegenstand der Erfindung ist daher ein Verfahren zur Herstellung eines Phosphorsäureesters, dadurch gekennzeichnet, dass eine Phosphorsäurekomponente ausgewählt aus Orthophosphorsäure und einem ihrer Derivate mit einer Alkoholkomponente, vorzugsweise Fettalkoholethoxylat, und Polyol mit mehr als 2 OH-Gruppen oder einem entsprechenden Alkoxylatgruppen-haltigen Polyol bei Temperaturen von 150 bis 250 °C, bevorzugt von 180 bis 240 °C und besonders bevorzugt von 200 bis 230 °C, umgesetzt wird, vorzugsweise ohne Zusatz eines Katalysators.

Geeignete Phosphorsäurederivate sind Polyphosphorsäure, Tetraphosphordecaoxid, Phosphoroxychlorid und Phosphorpentachlorid.

In einer bevorzugten Ausführungsform des erfindungsgemäßen Verfahrens wird als Phosphorsäurekomponente eine Substanz ausgewählt aus Orthophosphorsäure, Polyphosphorsäure und Tetraphosphordecaoxid, vorzugsweise Orthophosphorsäure, umgesetzt.

Die Veresterung wird bevorzugt soweit durchgeführt, dass im Wesentlichen neutrale erfindungsgemäße Phosphorsäureester vorliegen. Bevorzugt ist ein Umsetzungsgrad > 75 %, d. h. mehr als 75 % aller veresterbaren Funktionen der Phosphorsäure oder der Phorphorsäurederivate sind verestert. Besonders bevorzugt ist ein Umsetzungsgrad > 80 %, insbesondere bevorzugt > 85 %.

Die übrigen freien Valenzen am Phosphoratom können Säuregruppen sein, aber auch Gegenionen, gewählt aus Li⁺, Na⁺, K⁺, Mg⁺⁺, Ca⁺⁺, Al⁺⁺⁺, NH₄⁺, quaternären Ammoniumionen [HNR¹R²R³]⁺, wobei R¹, R² und R³ unabhängig voneinander Wasserstoff, eine lineare oder verzweigte Alkylgruppe mit 1 bis 22 Kohlenstoffatomen, eine lineare oder verzweigte, einfach oder mehrfach ungesättigte Alkenylgruppe mit 2 bis 22 Kohlenstoffatomen, eine lineare Mono-Hydroxyalkylgruppe mit 2 bis 10 Kohlenstoffatomen, bevorzugt eine Mono-Hydroxyethyl- oder Mono-Hydroxypropylgruppe, sowie eine lineare oder verzweigte Di-Hydroxyalkylgruppe mit 3 bis 10 Kohlenstoffatomen, sein können.

Der Neutralisationsgrad der unsubstituierten Phosphorvalenzen (P-OH) kann zwischen 0 und 100 % betragen.

Die erfindungsgemäßen Phosphorsäureester haben ein hervorragendes Verdickungsvermögen, sowohl für Zusammensetzungen auf wässriger oder wässrig-alkoholischer als auch für Zusammensetzungen auf wässrig-tensidischer Basis und tolerieren auch organische Lösungsmittel wie Alkohole.

Des Weiteren eignen sich die erfindungsgemäßen Phosphorsäureester auch in vorteilhafter Weise zur Herstellung von kosmetischen, pharmazeutischen und dermatologischen Zusammensetzungen.

Ein weiterer Gegenstand der Erfindung ist daher eine kosmetische, pharmazeutische oder dermatologische Zusammensetzung, dadurch gekennzeichnet, dass sie einen oder mehrere erfindungsgemäße Phosphorsäureester enthält.

Die erfindungsgemäßen Phosphorsäureester haben vielfältige Einsatzmöglichkeiten und eignen sich für den Einsatz in wässrigen, wässrig-alkoholischen und wässrig-tensidischen Zusammensetzungen, Emulsionen, Suspensionen, Dispersionen, Pudern und Sprays.

In einer bevorzugten Ausführungsform der Erfindung liegen die erfindungsgemäßen Zusammensetzungen, daher in Form von wässrigen, wässrig-alkoholischen oder wässrig-tensidischen Zusammensetzungen, Emulsionen, Suspensionen, Dispersionen, Pudern oder Sprays vor.

Besonders vorteilhaft ist, dass das Verdickungsvermögen der erfindungsgemäßen Phosphorsäureester auch in stark saurem Medium ausgeprägt ist.

Die erfindungsgemäßen Phosphorsäureester eignen sich deshalb besonders zur Verdickung und Stabilisierung von sauren kosmetischen Zusammensetzungen. Dies können z.B. kosmetische Zusammensetzungen sein, die Hydroxysäuren, wie Milchsäure, Glykolsäure, Salicylsäure, Zitronensäure oder Polyglykoldisäuren in freier oder teilweiser Neutralisation enthalten. Weiterhin können Formulierungen enthaltend Vitamin C oder Vitamin C-Derivate, Dihydroxyaceton oder Skinwhitening Actives wie Arbutin oder Glycyrrhetinsäure und deren Salze stabilisiert werden.

In einer weiteren bevorzugten Ausführungsform der Erfindung besitzen die erfindungsgemäßen Zusammensetzungen, einen pH-Wert von 2 bis 10, vorzugsweise von 2 bis 6, besonders bevorzugt von 2,5 bis 5 und insbesondere bevorzugt von 3 bis 4,5.

Die erfindungsgemäßen Phosphorsäureester eignen sich weiter hervorragend als Verdicker elektrolythaltiger Zusammensetzungen.

Als Elektrolyt zum Einsatz kommen anorganische Salze, bevorzugt Ammonium- oder Metallsalze, besonders bevorzugt von Halogeniden, beispielsweise CaCl₂, MgCl₂, LiCl, KCl und NaCl, Carbonaten, Hydrogencarbonaten, Phosphaten, Sulfaten, Nitraten, insbesondere bevorzugt Natriumchlorid, und/oder organische Salze, bevorzugt Ammonium- oder Metallsalze, besonders bevorzugt der Glykolsäure, Milchsäure, Zitronensäure, Weinsäure, Mandelsäure, Salicylsäure, Ascorbinsäure, Brenztraubensäure, Fumarsäure, Retinoesäure, Sulfonsäuren, Benzoesäure, Kojisäure, Fruchtsäure, Äpfelsäure, Gluconsäure Galacturonsäure.

Als Elektrolyt können die erfindungsgemäßen Zusammensetzungen auch Mischungen verschiedener Salze enthalten.

In einer weiteren bevorzugten Ausführungsform der Erfindung enthalten die erfindungsgemäßen Zusammensetzungen einen oder mehrere Elektrolyte.

Hierzu zählen auch wässrige Antiperspirant-Formulierungen enthaltend Aluminiumsalze, bevorzugt Aluminiumchlorohydrat oder Aluminium-Zirkonium-Komplexsalze.

Der Gehalt an dem einen oder den mehreren Elektrolyten, bezogen auf die gesamte erfindungsgemäße Zusammensetzung, ist vorzugsweise von 0,1 bis 20,0 Gew.-%, besonders bevorzugt von 0,2 bis 10,0 Gew.-% und insbesondere bevorzugt von 0,5 bis 5,0 Gew.-%.

Weiterhin sehr vorteilhaft ist, dass die erfindungsgemäßen Phosphorsäureester Zusammensetzungen, die Oxidationsmittel, vorzugsweise Wasserstoffperoxid, enthalten, beispielsweise Haarfärbemittel, sowohl verdicken als auch stabilisieren.

In einer weiteren bevorzugten Ausführungsform der Erfindung enthalten die erfindungsgemäßen Zusammensetzungen Wasserstoffperoxid oder Wasserstoffperoxid-freisetzende Substanzen und liegen vorzugsweise in der Form von Gelen oder Cremes vor.

Als Wasserstoffperoxid-freisetzende Substanzen kommen vorzugsweise in Betracht anorganische Persäuren, vorzugsweise Peroxoschwefelsäure, Peroxodischwefelsäure, Peroxocarbonate, sowie organische Persäuren, vorzugsweise Peressigsäure.

In einer weiteren bevorzugten Ausführungsform der Erfindung sind die erfindungsgemäßen Zusammensetzungen saure Wasserstoffperoxid-Bleichgele oder -cremes.

Die erfindungsgemäßen Phosphorsäureester sind in besonders vorteilhafter Weise zur Verdickung von kosmetischen, pharmazeutischen und dermatologischen Zusammensetzungen enthaltend ein oder mehrere Tenside geeignet. Hierbei handelt es sich vorzugsweise um Shampoos und Duschbäder.

In einer weiteren bevorzugten Ausführungsform der Erfindung enthalten die erfindungsgemäßen Zusammensetzungen ein oder mehrere Tenside.

In einer besonders bevorzugten Ausführungsform der Erfindung werden die erfindungsgemäßen Phosphorsäureester in Rinse-off Produkten, bevorzugt in Shampoos, Duschbädern, Duschgels und Schaumbädern, eingesetzt.

Die erfindungsgemäßen Phosphorsäureester sind in vorteilhafter Weise als Verdicker, Konsistenzgeber, Emulgator, Sensorikadditiv, Solubilisator, Dispergator, Suspendiermittel, Gleitmittel, Haftmittel und Stabilisator geeignet.

Ein weiterer Gegenstand der Erfindung betrifft daher die Verwendung eines oder mehrerer der erfindungsgemäßen Phosphorsäureester als Verdicker, Konsistenzgeber, Emulgator, Sensorikadditiv, Solubilisator, Dispergator, Suspendiermittel, Gleitmittel, Haftmittel oder Stabilisator, vorzugsweise die Verwendung als Verdicker.

In einer bevorzugten Ausführungsform der Erfindung werden die erfindungsgemäßen Phosphorsäureester zur Verdickung von kosmetischen, pharmazeutischen und dermatologischen Zusammensetzungen enthaltend ein oder mehrere Tenside, vorzugsweise von Shampoos oder Duschbädern verwendet.

Die erfindungsgemäßen Phosphorsäureester können als Verdicker für Mittel auf wässriger oder wässrig-alkoholischer Basis, beispielsweise Haargele, Feuchtigkeitsgele, Antiperspirantgele, Bleichgele, Konditioniermittel und Desinfektionsgele eingesetzt werden. Des Weiteren eignen sich die erfindungsgemäßen Phosphorsäureester als Stabilisator, Dispergiermittel und Konsistenzgeber für wässrig-tensidische Zubereitungen, beispielsweise Shampoos, Duschbäder, Duschgels und Schaumbäder und zur Verbesserung der Hautmilde und Hautkompatibilität.

Die verdickende Wirkung der erfindungsgemäßen Phosphorsäureester in wässrig-tensidischen Zusammensetzungen wird durch die Assoziation der hydrophoben Endgruppen mit den Tensidmicellen hervorgerufen und kann durch die Wahl der Ethoxylatendgruppen der erfindungsgemäßen Phosphorsäureester und durch die Wahl der Tenside gesteuert werden.

Die suspendierende bzw. dispergierende und stabilisierende Wirkung der erfindungsgemäßen Phosphorsäureester in wässrig-tensidischen Zusammensetzungen wird durch die Assoziation der hydrophoben Endgruppen und der in wässrig-tensidischen Mitteln unlöslichen flüssigen Komponenten, beispielsweise Öle und Silikonöle, bzw. der unlöslichen Feststoffkomponenten, beispielsweise Pigmente und Wirkstoffe wie Zink-Pyrithione, bedingt.

Die erfindungsgemäßen Phosphorsäureester eignen sich ebenso als Verdicker und Dispergiermittel, als Emulgatoren, Suspendiermittel mit verdickender Wirkung und Konsistenzgeber für Emulsionen und Suspensionen, wie Conditioner, sowie als Gleitmittel, Haftmittel, Verdicker, Dispergier- und Emulgiermittel dekorativer, feststoffhaltiger Zubereitungen. Dabei können auch Mischungen der erfindungsgemäßen Phosphorsäureester verwendet werden. Die emulgierende, stabilisierende und/oder konsistenzgebende Wirkung der erfindungsgemäßen Phosphorsäureester in Emulsionen wird durch eine Assoziation der hydrophoben Endgruppen untereinander, sowie durch eine Wechselwirkung der hydrophoben Endgruppen mit den hydrophoben Ölkomponenten verursacht bzw. verstärkt.

In Deodorant- oder Antiperspirant-Formulierungen enthaltend Aluminiumsalze, bevorzugt Aluminiumchlorohydrat oder Aluminium-Zirconium-Komplexsalze, zeigen die darin enthaltenen erfindungsgemäßen Phosphorsäureester den Vorteil, dass sie nach Anwendung der Formulierungen auf der Haut die Bildung weißer Rückstände auf der anschließend angezogenen Kleidung verringern.

Ein weiterer Gegenstand der vorliegenden Erfindung ist daher auch die Verwendung eines oder mehrerer erfindungsgemäßer Phosphorsäureester in Deodorant- oder Antiperspirant-Formulierungen, insbesondere in Deodorant- oder Antiperspirant-Formulierungen enthaltend Aluminiumsalze, bevorzugt Aluminiumchlorohydrat oder Aluminium-Zirconium-Komplexsalze, zur Verringerung der Bildung weißer Rückstände auf der Kleidung nach Anwendung der Deodorant- oder Antiperspirant-Formulierungen auf der Haut.

In einer weiteren bevorzugten Ausführungsform der Erfindung liegen die erfindungsgemäßen kosmetischen, pharmazeutischen oder darmatologischen Zusammensetzungen als Emulsionen vor.

Bei den Emulsionen kann es sich sowohl um Wasser-in-Öl-Emulsionen als auch um 01-in-Wasser-Emulsionen, Mikroemulsionen und multiple Emulsionen handeln.

Die Herstellung der Emulsionen kann in bekannter Weise, d.h. beispielsweise durch Heiß-, Heiß/Kalt- bzw. PIT-Emulgierung, erfolgen.

Der nichtwässrige Anteil der Emulsionen, der sich weitgehend aus dem Emulgator, dem Verdicker und dem Ölkörper zusammensetzt, liegt üblicherweise bei 5 bis 95 Gew.-%, vorzugsweise bei 15 bis 75 Gew.-%. Daraus folgt, dass die Emulsionen 5 bis 95 Gew.-%, vorzugsweise 25 bis 85 Gew.-% Wasser enthalten können, abhängig davon, ob Lotionen mit einer vergleichsweise niedrigen, oder Cremes und Salben mit hoher Viskosität hergestellt werden sollen.

In einer weiteren bevorzugten Ausführungsform der Erfindung werden die erfindungsgemäßen Phosphorsäureester in Leave-on Produkten, bevorzugt Hautpflegemitteln wie Tagescremes, Nachtcremes, Feuchtigkeitslotionen und -gelen, wässrigen Gelen, wie z.B. Gesichtstonern, Pflegecremes, Nährcremes, Bodylotions, Salben, Sonnenschutzmitteln, Lippenpflegemitteln, Antiperspirantien und Deodorantien, eingesetzt.

Des Weiteren eignen sie sich auch für tensidfreie, wässrige Zusammensetzungen und Emulsionen sowie für Haarkuren, Haarspülungen und Haargele, aber auch für Dauerwellenmittel, Haarfärbemittel, sowie für dekorative Kosmetika, beispielsweise make-ups, eye-shadows, Lippenstifte, Mascara und dergleichen.

Die erfindungsgemäßen Zusammensetzungen enthalten, bezogen auf die fertigen kosmetischen, pharmazeutischen oder dermatologischen Zusammensetzungen, vorzugsweise 0,01 bis 10,0 Gew.-%, besonders bevorzugt 0,1 bis 6,0 Gew.-% und insbesondere bevorzugt 0,5 bis 3,0 Gew.-% an den erfindungsgemäßen Phosphorsäureestern.

Die erfindungsgemäßen Zusammensetzungen können anionische, kationische, nichtionische, ampholytische Tenside und/oder Betaintenside enthalten.
Die Gesamtmenge der in den erfindungsgemäßen Mitteln (z.B. im Falle von Rinse-Off-Produkten) eingesetzten Tenside beträgt, bezogen auf die fertigen erfindungsgemäßen Zusammensetzungen, bevorzugt von 1,0 bis 70,0 Gew.-%, besonders bevorzugt von 5,0 bis 40,0 Gew.-% und insbesondere bevorzugt von 10,0 bis 35,0 Gew.-%.

Als anionische Tenside bevorzugt sind (C₁₀-C₂₂)-Alkyl- und Alkylen-carboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α-Sulfofettsäureester, Alkylbenzolsulfonate, Alkylphenolglykolethersulfonate, Sulfosuccinate, Sulfobernsteinsäurehalbester und -diester, Fettalkoholphosphate, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate und

Acylglycinate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Die Menge der anionischen Tenside in den erfindungsgemäßen Zusammensetzungen beträgt bevorzugt von 2,0 bis 30,0 Gew.-%, besonders bevorzugt von 5,0 bis 25,0 Gew.-% und insbesondere bevorzugt von 12,0 bis 22,0 Gew.-%, bezogen auf die fertigen Zusammensetzungen.

Bevorzugte kationische Tenside sind quartäre Ammonium-Salze, wie Di-(C₈-C₂₂)-Alkyl-dimethylammoniumchlorid oder -bromid, vorzugsweise Di-(C₈-C₂₂)-Alkyl-dimethylammoniumchlorid oder -bromid; (C₈-C₂₂)-Alkyl-dimethyl-ethylammoniumchlorid oder -bromid; (C₈-C₂₂)-Alkyl-trimethylammoniumchlorid oder -bromid, vorzugsweise Cetyltrimethylammoniumchlorid oder -bromid und (C₈-C₂₂)-Alkyl-trimethylammoniumchlorid oder -bromid; (C₁₀-C₂₄)-Alkyl-dimethylbenzyl-ammoniumchlorid oder -bromid, vorzugsweise (C₁₂-C₁₈)-Alkyl-dimethylbenzyl-ammoniumchlorid, (C₃-C₂₂)-Alkyl-dimethyl-hydroxyethylammoniumchlorid,- phosphat, -sulfat, -lactat, (C₈-C₂₂)-Alkylamidopropyltrimethylammoniumchlorid, -methosulfat, N,N-bis(2-C₈-C₂₂-Alkanoyl-oxyethyl)-dimethylammoniumchlorid, -methosulfat, N,N-bis(2-C₈-C₂₂-Alkanoyl-oxyethyl)hydroxyethyl-methyl-ammoniumchlorid, -methosulfat.

Die Menge der kationischen Tenside in den erfindungsgemäßen Zusammensetzungen beträgt bevorzugt von 0,1 bis 10,0 Gew.-%, besonders bevorzugt von 0,5 bis 7,0 Gew.-% und insbesondere bevorzugt von 1,0 bis 5,0 Gew.-%, bezogen auf die fertigen Zusammensetzungen.

Als nichtionische Tenside bevorzugt sind Fettalkoholethoxylate (Alkylpolyethylenglykole); Alkylphenolpolyethylenglykole; Fettaminethoxylate (Alkylaminopolyethylenglykole); Fettsäureethoxylate (Acylpolyethylenglykole); Polypropylenglykolethoxylate (Pluronics^{®}); Fettsäurealkanolamide, (Fettsäureamidpolyethylenglykole); Saccharoseester; Sorbitester und Sorbitanester und deren Polyglykolether, sowie C₈-C₂₂-Alkylpolyglucoside.

Die Menge der nichtionischen Tenside in den erfindungsgemäßen Zusammensetzungen (z. B. im Falle von Rinse-off-Produkten) liegt bevorzugt im Bereich von 1,0 bis 20,0 Gew.-%, besonders bevorzugt von 2,0 bis 10,0 Gew.-% und insbesondere bevorzugt von 3,0 bis 7,0 Gew.-%, bezogen auf die fertigen Zusammensetzungen.

Weiterhin können die erfindungsgemäßen Zusammensetzungen amphotere Tenside enthalten. Diese können beschrieben werden als Derivate langkettiger sekundärer oder tertiärer Amine, die über eine Alkylgruppe mit 8 bis 18 C-Atomen verfügen und bei denen eine weitere Gruppe substituiert ist mit einer anionischen Gruppe, die die Wasserlöslichkeit vermittelt, so z. B. mit einer Carboxyl-, Sulfat- oder Sulfonat-Gruppe. Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze; Geeignete weitere Tenside sind auch Aminoxide. Es sind dies Oxide tertiärer Amine mit einer langkettigen Gruppe von 8 bis 18 C-Atomen und zwei meist kurzkettigen Alkylgruppen mit 1 bis 4 C-Atomen. Bevorzugt sind hier beispielsweise die C₁₀- bis C₁₈-Alkyldimethylaminoxide, Fettsäureamidoalkyl-dimethylaminoxid.

Eine weitere bevorzugte Gruppe von Tensiden sind Betaintenside, auch zwitterionische Tenside genannt. Diese enthalten im selben Molekül eine kationische Gruppe, insbesondere eine Ammonium-Gruppe und eine anionische Gruppe, die eine Carboxylat-Gruppe, Sulfat-Gruppe oder Sulfonat-Gruppe sein kann. Geeignete Betaine sind vorzugsweise Alkylbetaine wie Coco-Betain oder Fettsäurealkylamidopropylbetaine, beispielsweise Kokosacylamidopropyldimethylbetain oder die C₁₂- bis C₁₈-Dimethylaminohexanoate bzw. die C₁₀- bis C₁₈-Acylamidopropandimetylbetaine.

Die Menge der amphoteren Tenside und/oder Betaintenside in den erfindungsgemäßen Zusammensetzungen beträgt bevorzugt von 0,5 bis 20,0 Gew.-% und besonders bevorzugt von 1,0 bis 10,0 Gew.-%, bezogen auf die fertigen Zusammensetzungen.

Bevorzugte Tenside sind Laurylsulfat, Laurethsulfat, Cocoamidopropylbetain, Alkylbetaine wie Coco-Betain, Natriumcocoylglutamat und Lauroamphoacetat.

In einer weiteren bevorzugten Ausführungsform der Erfindung enthalten die erfindungsgemäßen Zusammensetzungen zusätzlich noch als schaumverstärkende Mittel Co-Tenside aus der Gruppe der Alkylbetaine, Alkylamidobetaine, Aminopropionate, Aminoglycinate, Imidazoliniumbetaine und Sulfobetaine, Aminoxide, Fettsäurealkanolamide und Polyhydroxyamide.

Die erfindungsgemäßen Zusammensetzungen können als weitere Hilfs- und Zusatzstoffe Ölkörper, Silikonöle, Wachse, Emulgatoren, Co-Emulgatoren, Solubilisatoren, Stabilisatoren, kationische Polymere, Filmbildner, Verdicker, Gelierungsmittel, Überfettungsmittel, Rückfetter, antimikrobielle Wirkstoffe, biogene Wirkstoffe, Adstringentien, deodorierende Mittel, Sonnenschutzfilter, Antioxidantien, Feuchthaltemittel, Lösemittel, Farbstoffe, Duftstoffe, Perlglanzmittel, Trübungsmittel und/oder wasserlösliche Silikone enthalten.

Die Ölkörper können vorteilhafterweise ausgewählt werden aus den Gruppen der Triglyceride, natürlichen und synthetischen Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren oder aus der Gruppe der Alkylbenzoate, sowie natürlichen oder synthetischen Kohlenwasserstoffölen.

In Betracht kommen Triglyceride von linearen oder verzweigten, gesättigten oder ungesättigten, gegebenenfalls hydroxylierten, C₈-C₃₀-Fettsäuren, insbesondere pflanzliche Öle, wie Sonnenblumen-, Mais-, Soja-, Reis-, Jojoba-, Babusscu-, Kürbis-, Traubenkern-, Sesam-, Walnuss-, Aprikosen-, Orangen-, Weizenkeim-, Pfirsichkern-, Makadamia-, Avocado-, Süßmandel-, Wiesenschaumkraut-, Ricinusöl, Olivenöl, Erdnussöl, Rapsöl und Kokosnussöl, sowie synthetische Triglyceridöle, z. B. das Handelsprodukt Myritol^{®} 318. Auch gehärtete Triglyceride sind erfindungsgemäß bevorzugt. Auch Öle tierischen Ursprungs, beispielsweise Rindertalg, Perhydrosqualen, Lanolin können eingesetzt werden.

Eine weitere Klasse von bevorzugten Ölkörpern sind die Benzoesäureester von linearen oder verzweigten C₈₋₂₂-Alkanolen, z.B. die Handelsprodukte Finsolv^{®} SB (Isostearylbenzoat), Finsolv^{®} TN (C₁₂-C₁₅-Alkylbenzoat) und Finsolv^{®} EB (Ethylhexylbenzoat).

Eine weitere Klasse von bevorzugten Ölkörpern sind die Dialkylether mit insgesamt 12 bis 36 Kohlenstoffatomen, insbesondere mit 12 bis 24 Kohlenstoffatomen, wie z. B. Di-n-octylether (Cetiol^{®} OE), Di-n-nonylether, Di-n-decylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether, Di-3-ethyldecylether, tert.-Butyl-n-octylether, iso-Pentyl-n-octylether und 2-Methyl-pentyl-n-octylether sowie Di-tert.-butylether und Di-isopentylether.

Ebenso in Betracht kommen verzweigte gesättigte oder ungesättigte Fettalkohole mit 6-30 Kohlenstoffatomen, z. B. Isostearylalkohol, sowie Guerbetalkohole.

Eine weitere Klasse von bevorzugten Ölkörpern sind Hydroxycarbonsäurealkylester. Bevorzugte Hydroxycarbonsäurealkylester sind Vollester der Glykolsäure, Milchsäure, Apfelsäure, Weinsäure oder Zitronensäure. Weitere grundsätzlich geeignete Ester der Hydroxycarbonsäuren sind Ester der β-Hydroxypropionsäure, der Tartronsäure, der D-Gluconsäure, Zuckersäure, Schleimsäure oder Glucuronsäure. Als Alkoholkomponente dieser Ester eignen sich primäre, lineare oder verzweigte aliphatische Alkohole mit 8 bis 22 C-Atomen. Dabei sind die Ester von C₁₂-C₁₅-Fettalkoholen besonders bevorzugt. Ester dieses Typs sind im Handel erhältlich, z.B. unter dem Handelsnamen Cosmacol^{®} der EniChem, Augusta Industriale.

Eine weitere Klasse von bevorzugten Ölkörpern sind Dicarbonsäureester von linearen oder verzweigten C₂-C₁₀-Alkanolen, wie Di-n-butyladipat (Cetiol^{®} B), Di-(2-ethylhexyl)-adipat und Di-(2-ethylhexyl)-succinat sowie Diolester wie Ethylenglycol-dioleat, Ethylenglycol-di-isotridecanoat, Propylenglycol-di-(2-ethylhexanoat), Propylenglycol-di-isostearat, Propylenglycol-di-pelargonat, Butandiol-di-isostearat und Neopentylglycoldicaprylat sowie Di-isotridecylacelaat.

Ebenso bevorzugte Ölkörper sind symmetrische, unsymmetrische oder cyclische Ester der Kohlensäure mit Fettalkoholen, Glycerincarbonat oder Dicaprylylcarbonat (Cetiol^{®} CC).

Eine weitere Klasse von bevorzugten Ölkörpern sind die Ester von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertig linearen oder verzweigten C₂-C₆-Alkanolen.

Eine weitere Klasse von bevorzugten Ölkörpern sind Kohlenwasserstofföle, zum Beispiel solche mit linearen oder verzweigten, gesättigten oder ungesättigten C₇-C₄₀-Kohlenstoffketten, beispielsweise Vaseline, Dodecan, Isododecan, Cholesterol, Lanolin, synthetische Kohlenwasserstoffe wie Polyolefine, insbesondere Polyisobuten, hydriertes Polyisobuten, Polydecan, sowie Hexadecan, Isohexadecan, Paraffinöle, Isoparaffinöle, z. B. die Handelsprodukte der Permethyl^{®}-Serie, Squalan, Squalen, und alicyclische Kohlenwasserstoffe, z. B. das Handelsprodukt 1,3-Di-(2-ethyl-hexyl)-cyclohexan (Cetiol^{®}S), Ozokerit, und Ceresin.

An Silikonölen bzw. -wachsen stehen vorzugsweise zur Verfügung Dimethylpolysiloxane und Cyclomethicone, Polydialkylsiloxane R₃SiO(R₂SiO)ₓSiR₃, wobei R für Methyl oder Ethyl, besonders bevorzugt für Methyl, steht und x für eine Zahl von 2 bis 500 steht, beispielsweise die unter den Handelsnamen VICASIL (General Electric Company), DOW CORNING 200, DOW CORNING 225, DOW CORNING 200 (Dow Corning Corporation), erhältlichen Dimethicone, sowie die unter SilCare^{®} Silicone 41 M65, SilCare^{®} Silicone 41 M70, SilCare^{®} Silicone 41 M80 (Clariant GmbH) erhältlichen Dimethicone, Stearyldimethylpolysiloxan, C₂₀-C₂₄-Alkyl-dimethylpolysiloxan, C₂₄-C₂₈-Alkyl-dimethylpolysiloxan, aber auch die unter SilCare^{®} Silicone 41 M40, SilCare^{®} Silicone 41 M50 (Clariant GmbH) erhältlichen Methicone, weiterhin Trimethylsiloxysilicate [(CH₂)₃SiO)_{1/2}]ₓ[SiO₂]_{y}, wobei x für eine Zahl von 1 bis 500 und y für eine Zahl von 1 bis 500 steht, Dimethiconole R₃SiO[R₂SiO]ₓSiR₂OH und HOR₂SiO[R₂SiO]ₓSiR₂OH, wobei R für Methyl oder Ethyl und x für eine Zahl bis zu 500 steht, Polyalkylarylsiloxane, beispielsweise die unter den Handelsbezeichnungen SF 1075 METHYLPHENYL FLUID (General Electric Company) und 556 COSMETIC GRADE PHENYL TRIMETHICONE FLUID (Dow Corning Corporation) erhältlichen Polymethylphenylsiloxane, Polydiarylsiloxane, Silikonharze, cyclische Silikone und amino-, fettsäure-, alkohol-, polyether-, epoxy-, fluor- und/oder alkylmodifizierte Silikonverbindungen, sowie Polyethersiloxan-Copolymere.

Die erfindungsgemäßen Zusammensetzungen können Wachse, beispielsweise Paraffinwachse, Mikrowachse und Ozokerit, Bienenwachs und ihre Teilfraktionen sowie der Bienenwachsderivate, Wachse aus der Gruppe der homopolymeren Polyethylene oder Coplymere der α-Olefine, sowie natürliche Wachse wie Reiswachs, Candellilawachs, Carnaubawachs, Japanwachs oder Schellackwachs enthalten.

Als Emulgatoren, Co-Emulgatoren und Solubilisatoren können nichtionische, anionische, kationische oder amphotere oberflächenaktive Verbindungen eingesetzt werden.

Als nichtionogene oberflächenaktive Verbindungen kommen vorzugsweise in Betracht:
Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und gegebenenfalls deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z. B. Polyglycerinpolyricinoleat und Polyglycerinpoly-12-hydroxystearat. Ebenfalls vorzugsweise geeignet sind ethoxylierte Fettamine, Fettsäureamide, Fettsäurealkanolamide und Gemische von Verbindungen aus mehreren dieser Substanzklassen.

Als ionogene Co-Emulgatoren eignen sich z. B. anionische Emulgatoren, wie mono-, di- oder tri-Phosphorsäureester, Seifen (z. B. Natriumstearat), Fettalkoholsulfate aber auch kationische Emulgatoren wie mono-, di- und tri-Alkylquats und deren polymere Derivate.

An amphoteren Emulgatoren stehen vorzugsweise zur Verfügung Alkylaminoalkylcarbonsäuren, Betaine, Sulfobetaine und Imidazolinderivate.

Besonders bevorzugt zum Einsatz kommen Fettalkoholethoxylate, gewählt aus der Gruppe der ethoxylierten Stearylalkohole, Isostearylalkohole, Cetylalkohole, Isocetylalkohole, Oleylalkohole, Laurylalkohole, Isolaurylalkohole und Cetylstearylalkohole, insbesondere Polyethylenglycol(13)stearylether, Polyethylenglycol(14)stearylether, Polyethylenglycol(15)stearylether, Polyethylenglycol(16)stearylether, Polyethylenglycol(17)stearylether, Polyethylenglycol(18)stearylether, Polyethylenglycol(19)stearylether, Polyethylenglycol(20)stearylether, Polyethylenglycol(12)isostearylether, Polyethylenglycol(13)isostearylether, Polyethylenglycol(14)isostearylether, Polyethylenglycol(15)isostearylether, Polyethylenglycol(16)isostearylether, Polyethylenglycol(17)isostearylether, Polyethylenglycol(18)isostearylether, Polyethylenglycol(19)isostearylether, Polyethylenglycol(20)isostearylether, Polyethylenglycol(13)cetylether, Polyethylenglycol(14)cetylether, Polyethylenglycol(15)cetylether, Polyethylenglycol(16)cetylether, Polyethylenglycol(17)cetylether, Polyethylenglycol(18)cetylether, Polyethylenglycol(19)cetylether, Polyethylenglycol(20)cetylether, Polyethylenglycol(13)isocetylether, Polyethylenglycol(14)isocetylether, Polyethylenglycol(15)isocetylether, Polyethylenglycol(16)isocetylether, Polyethylenglycol(17)isocetylether, Polyethylenglycol(18)isocetylether, Polyethylenglycol(19)isocetylether, Polyethylenglycol(20)isocetylether, Polyethylenglycol(12)oleylether, Polyethylenglycol(13)oleylether, Polyethylenglycol(14)oleylether, Polyethylenglycol(15)oleylether, Polyethylenglycol(12)laurylether, Polyethylenglycol(12)isolaurylether, Polyethylenglycol(13)cetylstearylether, Polyethylenglycol(14)cetylstearylether, Polyethylenglycol(15)cetylstearylether, Polyethylenglycol(16)cetylstearylether, Polyethylenglycol(17)cetylstearylether, Polyethylenglycol(18)cetylstearylether, Polyethylenglycol(19)cetylstearylether.

Ebenso bevorzugt sind Fettsäureethoxylate, gewählt aus der Gruppe der ethoxylierten Stearate, Isostearate und Oleate, insbesondere Polyethylenglycol(20)stearat, Polyethylenglykol(21)stearat, Polyethylenglykol(22)stearat, Polyethylenglykol(23)stearat, Polyethylenglykol(24)stearat, Polyethylenglykol(25)stearat, Polyethylenglykol(12)isostearat, Polyethylenglykol(13)isostearat, Polyethylenglykol(14)isostearat, Polyethylenglykol(15)isostearat, Polyethylenglykol(16)isostearat, Polyethylenglykol(17)isostearat, Polyethylenglykol(18)isostearat, Polyethylenglykol(19)isostearat, Polyethylenglykol(20)isostearat, Polyethylenglykol(21)isostearat, Polyethylenglykol(22)isostearat, Polyethylenglykol(23)isostearat, Polyethylenglykol(24)isostearat, Polyethylenglykol(25)isostearat, Polyethylenglykol(12)oleat, Polyethylenglykol(13)oleat, Polyethylenglykol(14)oleat, Polyethylenglykol(15)oleat, Polyethylenglykol(16)oleat, Polyethylenglykol(17)oleat, Polyethylenglykol(18)oleat, Polyethylenglykol(19)oleat, Polyethylenglykol(20)oleat.

Als ethoxylierte Alkylethercarbonsäure oder deren Salze kann vorteilhafterweise das Natrium Laureth-11-carboxylat verwendet werden.

Als ethoxylierte Triglyceride können vorteilhaft Polyethylenglykol(60)Evening Primose Glyceride verwendet werden.

Weiterhin ist es von Vorteil, die Polyethylenglykolglycerinfettsäureester aus der Gruppe Polyethylenglykol(20)glyceryllaurat, Polyethylenglykol(6)glycerylcaprat/caprinat, Polyethylenglykol(20)glyceryloleat, Polyethylenglykol(20)glycerylisostearat und Polyethylenglykol(18)glyceryloleat/cocoat zu wählen.

Unter den Sorbitanestern eignen sich besonders
Polyethylenglykol(20)sorbitanmonolaurat,
Polyethylenglycol(20)sorbitanmonostearat,
Polyethylenglycol(20)sorbitanmonoisostearat,
Polyethylenglycol(20)sorbitanmonopalmitat,
Polyethylenglycol(20)sorbitanmonooleat.

Besonders vorteilhafte Coemulgatoren sind Glycerylmonostearat, Glycerylmonooleat, Diglycerylmonostearat, Glycerylisosterat, Polyglyceryl-3-oleat, Polyglyceryl-3-diisostearat, Polyglyceryl-4-isostearat, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Glycoldistearat und Polyglyceryl-3-dipolyhydroxystearat, Sorbitanmonoisostearat, Sorbitanstearat, Sorbitanoleat, Saccharosedistearat, Lecithin, PEG-7-hydriertes Ricinusöl, Cetylalkohol, Stearylalkohol, Behenylalkohol, Isobehenylalkohol und Polyethylenglycol(2)stearylether (Steareth-2), Alkylmethiconcopolyole und Alkyl-Dimethiconcopolyole, insbesondere Cetyldimethiconcopolyol, Laurylmethiconcopolyol.

Die erfindungsgemäßen Zusammensetzungen können einen oder mehrere der Emulgatoren, Co-Emulgatoren oder Solubilisatoren in Mengen von 0,1 bis 20,0 Gew.-%, vorzugsweise 1,0 bis 15,0 Gew.-% und besonders bevorzugt 3,0 bis 10,0 Gew.-%, bezogen auf die fertigen Zusammensetzungen, enthalten.

Als Stabilisatoren können Metallsalze von Fettsäuren, wie z. B. Magnesium-, Aluminium- und/oder Zinkstearat eingesetzt werden, bevorzugt in Mengen von 0,1 bis 10,0 Gew.-%, vorzugsweise von 0,5 bis 8,0 Gew.-% und besonders bevorzugt von 1,0 bis 5,0 Gew.-%, bezogen auf die fertigen Zusammensetzungen.

Als kationische Polymere eignen sich die unter der INCl-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVP-dimethylaminoethylmethacrylat-Copolymer, Guarhydroxypropyltriammonium-chloride, sowie Calciumalginat und Ammoniumalginat. Des Weiteren können eingesetzt werden kationische Cellulosederivate; kationische Stärke; Copolymere von Diallylammoniumsalzen und Acrylamiden; quaternierte Vinylpyrrolidon/Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z. B. Amidomethicone; Copolymere der Adipinsäure und Dimethylaminohydroxypropyldiethylentriamin; Polyaminopolyamid und kationische Chitinderivate, wie beispielsweise Chitosan.

Die erfindungsgemäßen Zusammensetzungen können einen oder mehrere der oben genannten kationischen Polymere in Mengen von 0,1 bis 5,0 Gew.-%, vorzugsweise von 0,2 bis 3,0 Gew.-% und besonders bevorzugt von 0,5 bis 2,0 Gew.-%, bezogen auf die fertigen Zusammensetzungen, enthalten.

Des Weiteren können die erfindungsgemäßen Zusammensetzungen Filmbildner enthalten, die je nach Anwendungszweck ausgewählt sind aus Salzen der Phenylbenzimidazolsulfonsäure, wasserlöslichen Polyurethanen, beispielsweise C₁₀-Polycarbamylpolyglycerylester, Polyvinylalkohol,
Polyvinylpyrrolidoncopolymeren, beispielsweise Vinylpyrrolidon/Vinylacetatcopolymer, wasserlöslichen
Acrylsäurepolymeren/Copolymeren bzw. deren Estern oder Salzen, beispielsweise

Partialestercopolymere der Acryl/Methacrylsäure, wasserlösliche Cellulose, beispielsweise Hydroxymethylcellulose, Hydroxyethylcellulose, Hydroxypropylcellulose, wasserlöslichen Quaterniums, Polyquaterniums, Carboxyvinyl-Polymeren, wie Carbomere und deren Salze, Polysacchariden, beispielsweise Polydextrose und Glucan, Vinylacetat/Crotonat, beispielsweise unter dem Handelsnamen Aristoflex^{®} A 60 (Clariant) erhältlich.

Die erfindungsgemäßen Zusammensetzungen können einen oder mehrere Filmbildner in Mengen von 0,1 bis 10,0 Gew.-%, vorzugsweise von 0,2 bis 5,0 Gew.-% und besonders bevorzugt von 0,5 bis 3,0 Gew.-%, bezogen auf die fertigen Zusammensetzungen, enthalten.

Die gewünschte Viskosität der Zusammensetzungen kann durch Zugabe von Verdickern und Gelierungsmittel eingestellt werden. In Betracht kommen vorzugsweise Celluloseether und andere Cellulosederivate (z. B. Carboxymethylcellulose, Hydroxyethylcellulose), Gelatine, Stärke und Stärkederivate, Natriumalginate, Fettsäurepolyethylenglykolester, Agar-Agar, Traganth oder Dextrinderivate, insbesondere Dextrinester. Des Weiteren eignen sich Metallsalze von Fettsäuren, bevorzugt mit 12 bis 22 C-Atomen, beispielsweise Natriumstearat, Natriumpalmitat, Natriumlaurat, Natriumarachidate, Natriumbehenat, Kaliumstearat, Kaliumpalmitat, Natriummyristat, Aluminiummonostearat, Hydroxyfettsäuren, beispielsweise 12-Hydroxystearinsäure, 16-Hydroxyhexadecanoylsäure; Fettsäureamide; Fettsäurealkanolamide; Dibenzalsorbit und alkohollösliche Polyamide und Polyacrylamide oder Mischungen solcher. Weiterhin können vernetzte und unvernetzte Polyacrylate wie Carbomer, Natriumpolyacrylate oder sulfonsäurehaltige Polymere wie Ammoniumacryloyldimethyltaurate/VP-Copolymer Verwendung finden.

Bevorzugt enthalten die erfindungsgemäßen Zusammensetzungen 0,01 bis 20,0 Gew.-%, besonders bevorzugt 0,1 bis 10,0 Gew.-%, insbesondere bevorzugt 0,2 bis 3,0 Gew.-% und ganz besonders bevorzugt 0,4 bis 2,0 Gew.-% an Verdickern bzw. Geliermitteln, bezogen auf die fertigen erfindungsgemäßen Zusammensetzungen.

Als Überfettungsmittel können vorzugsweise Lanolin und Lecithin, nicht ethoxylierte und polyethoxylierte oder acylierte Lanolin- und Lecithinderivate, Polyolfettsäureester, Mono-, Di- und Triglyceride und/oder Fettsäurealkanolamide, wobei die letzteren gleichzeitig als Schaumstabilisatoren dienen, verwendet werden, die bevorzugt in Mengen von 0,01 bis 10,0 Gew.-%, besonders bevorzugt von 0,1 bis 5,0 Gew.-% und insbesondere bevorzugt von 0,5 bis 3,0 Gew.-% eingesetzt werden, bezogen auf die fertigen erfindungsgemäßen Zusammensetzungen.

An antimikrobiellen Wirkstoffen kommen Cetyltrimethylammoniumchlorid, Cetylpyridiniumchlorid, Benzethoniumchlorid, Diisobutylethoxyethyldimethylbenzylammoniumchlorid, Natrium N-Laurylsarcosinat, Natrium-N-Palmethylsarcosinat, Lauroylsarcosin, N-Myristoylglycin, Kalium-N-Laurylsarcosin, Trimethylammoniumchlorid, Natriumaliuminiumchlorohydroxylactat, Triethylcitrat, Tricetylmethylammoniumchlorid, 2,4,4'-Trichloro-2'-hydroxydiphenylether (Triclosan), Phenoxyethanol, 1,5-Pentandiol, 1,6-Hexandiol, 3,4,4'-Trichlorocarbanilid (Triclocarban), Diaminoalkylamid, beispielsweise L-Lysinhexadecylamid, Citratschwermetallsalze, Salicylate, Piroctose, insbesondere Zinksalze, Pyrithione und deren Schwermetallsalze, insbesondere Zinkpyrithion, Zinkphenolsulfat, Farnesol, Ketoconazol, Oxiconazol, Bifonazole, Butoconazole, Cloconazole, Clotrimazole, Econazole, Enilconazole, Fenticonazole, Isoconazole, Miconazole, Sulconazole, Tioconazole, Fluconazole, Itraconazole, Terconazole, Naftifine und Terbinafine, Selendisulfid und Octopirox^{®}, lodopropynylbutylcarbamat, Methylchloroisothiazolinon, Methylisothiazolinon, Methyldibromo Glutaronitril, AgCl, Chloroxylenol, Na-Salz von Diethylhexylsulfosuccinat, Natriumbenzoat, sowie Phenoxyethanol, Benzylalkohol, Phenoxyisopropanol, Parabene, bevorzugt Butyl-, Ethyl-, Methyl- und Propylparaben, sowie deren Na-Salze, Pentandiol 1,2-Octandiol, 2-Bromo-2-Nitropropan-1,3-diol, Ethylhexylglycerin, Benzylalkohol, Sorbinsäure, Benzoesäure, Milchsäure, Imidazolidinylharnstoff, Diazolidinylharnstoff, Dimethyloldimethylhydantoin (DMDMH), Na-Salz von Hydroxymethylglycinat, Hydroxyethylglycin der Sorbinsäure und Kombinationen dieser Wirksubstanzen zum Einsatz.

Die erfindungsgemäßen Zusammensetzungen enthalten die antimikrobiellen Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen erfindungsgemäßen Zusammensetzungen.

Die erfindungsgemäßen Zusammensetzungen können des Weiteren biogene Wirkstoffe, ausgewählt aus Pflanzenextrakten, wie beispielsweise Aloe Vera, sowie Lokalanästhetika, Antibiotika, Antiphlogistika, Antiallergica, Corticosteroide, Sebostatika, Bisabolol^{®}, Allantoin, Phytantriol^{®}, Proteine, Vitamine ausgewählt aus Niacin, Biotin, Vitamin B2, Vitamin B3, Vitamin B6, Vitamin B3 Derivaten (Salzen, Säuren, Estern, Amiden, Alkoholen), Vitamin C und Vitamin C Derivaten (Salzen, Säuren, Estern, Amiden, Alkoholen), bevorzugt als Natriumsalz des Monophosphorsäureesters der Ascorbinsäure oder als Magnesiumsalz des Phosphorsäureesters der Ascorbinsäure, Tocopherol und Tocopherolacetat, sowie Vitamin E und/oder dessen Derivate enthalten.

Die erfindungsgemäßen Zusammensetzungen können biogene Wirkstoffe bevorzugt in Mengen von 0,001 bis 5,0 Gew.-%, besonders bevorzugt von 0,01 bis 3,0 Gew.-% und insbesondere bevorzugt von 0,1 bis 2,0 Gew.-%, bezogen auf die fertigen Zusammensetzungen, enthalten.

Die erfindungsgemäßen Zusammensetzungen können Adstringentien, bevorzugt Magnesiumoxid, Aluminiumoxid, Titandioxid, Zirkondioxid und Zinkoxid, Oxidhydrate, bevorzugt Aluminiumoxidhydrat (Böhmit) und Hydroxide, bevorzugt von Calcium, Magnesium, Aluminium, Titan, Zirkon oder Zink, sowie Aluminiumchlorohydrate bevorzugt in Mengen von 0 bis 50,0 Gew.-%, besonders bevorzugt in Mengen von 0,01 bis 10,0 Gew.-% und insbesondere bevorzugt in Mengen von 0,1 bis 10,0 Gew.-% enthalten. Als deodorierende Stoffe bevorzugt sind Allantoin und Bisabolol. Diese werden vorzugsweise in Mengen von 0,0001 bis 10,0 Gew.-% eingesetzt.

Die erfindungsgemäßen Zusammensetzungen können als Pigmente/Mikropigmente sowie als Sonnenschutzfilter mikrofeines Titandioxid, Glimmer-Titanoxid, Eisenoxide, Glimmer-Eisenoxid, Zinkoxid, Siliciumoxide, Ultramarinblau, Chromoxide enthalten.

Die erfindungsgemäßen Zusammensetzungen können Sonnenschutzfilter enthalten, vorzugsweise ausgewählt aus 4-Aminobenzoesäure, 3-(4'-Trimethylammonium)-benzyliden-boran-2-on-methylsulfat, Camphor Benzalkonium Methosulfat, 3,3,5-Trimethyl-cyclohexylsalicylat, 2-Hydroxy-4-methoxybenzophenon, 2-Phenylbenzimidazol-5-sulfonsäure und ihre Kalium-, Natrium- und Triethanolaminsalze, 3,3'-(1,4-Phenylendimethin)-bis-(7,7-dimethyl-2-oxobicyclo[2.2.1]-heptan-1-methansulfonsäure) und ihre Salze, 1-(4-tert.-Butylphenyl)-3-(4-methoxyphenyl)propan-1,3-dion, 3-(4'-Sulfo)-benzyliden-bornan-2-on und seine Salze, 2-Cyan-3,3-diphenyl-acrylsäure-(2-ethylhexylester), Polymere von N-[2(und 4)-(2-oxoborn-3-ylidenmethyl)benzyl]-acrylamid, 4-Methoxy-zimtsäure-2-ethyl-hexylester, ethoxyliertes Ethyl-4-amino-benzoat, 4-Methoxy-zimtsäure-isoamylester, 2,4,6-Tris-[p-(2-ethylhexyloxycarbonyl)anilino]-1,3,5-triazin, 2-(2H-benzotriazol-2-yl)-4-methyl-6-(2-methyl-3-(1,3,3,3-tetramethyl-1-(trimethylsilyloxy)-disiloxanyl)-propyl)phenol, 4,4'-[(6-[4-((1,1-dimethylethyl)-amino-carbonyl)phenylamino]-1,3,5-triazin-2,4-yl)diimino]bis-(benzoesäure-2-ethylhexylester), Benzophenon-3, Benzophenon-4 (Säure), 3-(4'-Methylbenzyliden)-D,L-Campher, 3-Benzyliden-Campher, Salicylsäure-2-ethylhexylester, 4-Dimethylaminobenzoesäure-2-ethylhexylester, Hydroxy-4-methoxy-benzophenon-5-sulfonsäure (Sulfisobenzonum) und das Natriumsalz, 4-Isopropylbenzylsalicylat, N,N,N-Trimethyl-4-(2-oxoborn-3-ylidenemethyl) anilium methyl sulphate, Homosalate (INN), Oxybenzone (INN), 2-Phenylbenzimidazole-5-sulfonsäure und ihre Natrium-, Kalium-, und Triethanolaminsalze, Octylmethoxyzimtsäure, Isopentyl-4-methoxyzimtsäure, Isoamyl-p-methoxyzimtsäure, 2,4,6-Trianilino-(p-carbo-2'-ethylhexyl-1'-oxy)-1,3,5-triazine (Octyl triazone) Phenol, 2-2(2H-benzotriazol-2-yl)-4-methyl-6-(2-methyl-3-(1,3,3,3-tetramethyl-1-(trimethylsilyl)oxy)-disiloxanyl)propyl (Drometrizole Trisiloxane) benzoesäure, 4,4-((6-(((1,1-dimethylethyl)amino)carbonyl)phenyl)amino)-1,3,5-triazine-2,4-diyl)diimino)bis,bis(2-ethylhexyl)ester) benzoesäure, 4,4-((6-(((1,1-dimethytethyl)amino)-carbonyl)phenyl)amino)-1,3,5-triazine-2,4-diyl)diimino)bis,bis(2-ethylhexyl)ester), 3-(4'-Methylbenzyliden)-D,L-campher (4-Methylbenzyliden Camphor), Benzyliden-camphor-sulfonsäure, Octocrylen, Polyacrylamidomethyl-Benzyliden-Camphor, 2-Ethylhexyl salicylat (Octyl Salicylat), 4-Dimethyl-aminobenzoesäureethyl-2-hexylester (octyl dimethyl PABA), PEG-25 PABA, 2-Hydroxy-4-methoxybenzo-phenone-5-sulfonsäure (Benzophenone-5) und das Na-Salz, 2,2'-Methylen-bis-6-(2H-benzotriazol-2yl)-4-(tetramethyl-butyl)-1,1,3,3-phenol, Natriumsalz von 2-2'-bis-(1,4-phenylen)1H-benzimidazole-4,6-disulfonsäure, (1,3,5)-Triazine-2,4-bis((4-(2-ethyl-hexyloxy)-2-hydroxy)-phenyl)-6-(4-methoxyphenyl), 2-Ethylhexyl-2-cyano-3,3-diphenyl-2-propenoat, Glyceryl octanoat, Di-p-methoxyzimtsäure, p-Amino-benzoesäure und deren Ester, 4-tert-Butyl-4'-methoxydibenzoylmethan, 4-(2-β-Glucopyranoxy)propoxy-2-hydroxybenzophenon, Octyl Salicylat, Methyl-2,5-diisopropylzimtsäure, Cinoxat, Dihydroxy-dimethoxybenzophenon, Dinatriumsalz von 2,2'-Dihydroxy-4,4'-dimethoxy-5,5'-disulfobenzophenon, Dihydroxybenzophenon, 1,3,4-Dimethoxyphenyl-4,4-dimethyl-1,3-pentanedion, 2-Ethylhexyl-dimethoxybenzyliden-dioxoimidazolidinpropionat, Methylen-Bis-Benztriazolyl Tetramethylbutylphenol, Phenyldibenzimidazoltetrasulfonat, Bis-Ethylhexyloxyphenol-Methoxyphenol-Triazin, Tetrahydroxybenzophenone, Terephthalylidendicampher-sulfonsäure, 2,4,6-tris[4,2-Ethylhexyloxycarbonyl)anilino]-1,3,5-triazin, Methyl-bis(trimethylsiloxy)silyl-isopentyl trimethoxy-zimtsäure, Amyl-p-dimethylaminobenzoat, Amyl-p-dimethylamino benzoat, 2-Ethylhexyl-p-dimethylaminobenzoat, Isopropyl-p-methoxyzimtsäure/ Diisopropylzimtsäureester, 2-Ethylhexyl-p-methoxyzimtsäure, 2-Hydroxy-4-methoxy benzophenon, 2-Hydroxy-4-methoxybenzophenon-5-sulfsäure und das Trihydrat, sowie 2-Hydroxy-4-methoxybenzophenon-5-sulfonat Natriumsalz und Phenyl-benzimidazol-sulfonsäure.

Die Menge der vorgenannten Sonnenschutzfilter (eine oder mehrere Verbindungen) in den erfindungsgemäßen Zusammensetzungen beträgt vorzugsweise von 0,001 bis 30,0 Gew.-%, besonders bevorzugt von 0,05 bis 20,0 Gew.-% und insbesondere bevorzugt von 1,0 bis 10,0 Gew.-%, bezogen auf das Gesamtgewicht der fertigen Zusammensetzung.

Die erfindungsgemäßen Zusammensetzungen können Antioxidantien enthalten, vorzugsweise ausgewählt aus Aminosäuren (z. B. Glycin, Histidin, Tyrosin, Tryptophan) und deren Derivaten, Imidazolen (z. B. Urocaninsäure) und deren Derivaten, Peptiden wie D,L-Carnosin, D-Carnosin, L-Carnosin und deren Derivaten (z. B. Anserin), Carotinoiden, Carotinen (z. B. α-Carotin, β-Carotin, Lycopin) und deren Derivaten, Chlorogensäure und deren Derivaten, Liponsäure und deren Derivaten (z. B. Dihydroliponsäure), Aurothioglucose, Propylthiouracil und anderen Thiolen (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (z. B. Ester, Ether, Peptide, Lipide, Nukleotide, Nukleoside und Salze), sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen, ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Äpfelsäure), Huminsäure, Gallensäure, Gallenextrakten, Bilirubin, Biliverdin, EDTA, EGTA und deren Derivaten, ungesättigten Fettsäuren und deren Derivaten (z. B. γ-Linolensäure, Linolsäure, Ölsäure), Folsäure und deren Derivaten, Ubichinon und Ubichinol und deren Derivaten, Vitamin C und Derivaten (z. B. Ascorbylpalmitat, Mg-Ascorbylphosphat, Ascorbylacetat), Tocopherolen und Derivaten (z. B. Vitamin E-acetat), Vitamin A und Derivaten (Vitamin A-palmitat) sowie Koniferylbenzoat des Benzoeharzes, Rutinsäure und deren Derivaten, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z. B. ZnO, ZnSO₄), Selen und dessen Derivaten (z.B. Selenmethionin), Stilbenen und deren Derivaten (z. B. Stilbenoxid, trans-Stilbenoxid), Superoxid-Dismutase und den erfindungsgemäß geeigneten Derivaten (Salzen, Estern, Ethern, Zuckern, Nukleotiden, Nukleosiden, Peptiden und Lipiden) dieser genannten Stoffe.

Die Antioxidantien können die Haut und das Haar vor oxidativer Beanspruchung schützen. Bevorzugte Antioxidantien sind dabei Vitamin E und dessen Derivate sowie Vitamin A und dessen Derivate.

Die Menge des einen oder der mehreren Antioxidantien in den erfindungsgemäßen Zusammensetzungen beträgt vorzugsweise von 0,001 bis 30,0 Gew.-%, besonders bevorzugt von 0,05 bis 20,0 Gew.-% und insbesondere bevorzugt von 1,0 bis 10,0 Gew.-%, bezogen auf das Gesamtgewicht der Zusammensetzung.

Des Weiteren können Feuchthaltemittel, ausgewählt aus dem Natriumsalz von 2-Pyrrolidone-5-carboxylat (NaPCA), Guanidin; Glycolsäure und deren Salzen, Milchsäure und deren Salzen, Glucosamine und deren Salzen, Lactamidmonoethanolamin, Acetamidmonoethanolamin, Harnstoff, Hydroxysäuren, Panthenoi und dessen Derivaten, beispielsweise D-Panthenol (R-2,4-Dihydroxy-N-(3-hydroxypropyl)-3,3-dimethylbutamid), D,L-Panthenol, Calciumpantothenat, Panthetin, Pantothein, Panthenylethylether, Isopropylpalmitat, Glycerin und/oder Sorbitol eingesetzt werden, bevorzugt in Mengen von 0,1 bis 15,0 Gew.-% und besonders bevorzugt von 0,5 bis 5,0 Gew.-%, bezogen auf die fertigen Zusammensetzungen.

Zusätzlich können die erfindungsgemäßen Zusammensetzungen organische Lösungsmittel enthalten. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45,0 Gew.-% und von Polyethylenglykol mit einer relativen Molekülmasse zwischen 400 und 600 in Mengen von 5,0 bis 25,0 Gew.-% bevorzugt. Weitere geeignete Lösungsmittel sind beispielsweise Triacetin (Glycerintriacetat) und 1-Methoxy-2-propanol.

Die in den erfindungsgemäßen Zusammensetzungen enthaltenen Farbstoffe- und -pigmente, sowohl organische als auch anorganische Farbstoffe sind aus der entsprechenden Positivliste der Kosmetikverordnung bzw. der EG-Liste kosmetischer Färbemittel ausgewählt.

| Chemische oder sonstige Bezeichnung | CIN | Farbe |
|---|---|---|
| Pigment Green | 10006 | grün |
| Acid Green 1 | 10020 | grün |
| 2,4-Dinitrohydroxynaphtalin-7-sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro-1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'-Methoxy-5'-sulfosäurediethylamid-1'-phenylazo)-3-hydroxy-5"-chloro-2",4"-dimethoxy-2-naphthoesäureanilid | 12490 | rot |
| Disperse Yellow 16 | 12700 | gelb |
| 1-(4-Sulfo-1-phenylazo)-4-amino-brezol-sulfosäure | 13015 | gelb |
| 2,4-Dihydroxy-azobenzol-4'-sulfosäure | 14270 | orange |
| 2-(2,4-Dimethylphenylazo-5-sulfosäurse)-1-hydroxynaphthalin-4-sulfosäure | 14700 | rot |
| 2-(4-Sulfo-1-naphthylazo)-1-naphthol-4-sulfosäure | 14720 | rot |
| 2-(6-Sulfo-2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2-hydroxynaphthalin | 15525 | rot |
| 1-(3-Methyl-phenylazo-4-sulfosäure)-2-hydroxynaphthalin | 15580 | rot |
| 1-(4',(8')-Sulfosäurenaphthylazoy)-2-hydroxynaphthalin | 15620 | rot |
| 2-Hydroxy-1,2'-azonaphthalin-1'-sulfosäure | 15630 | rot |
| 3-Hydroxy-4-phenylazo-2-naphthylcarbonsäure | 15800 | rot |
| 1-(2-Sulfo-4-methyl-1-phenylazo)-2-naphthylcarbonsäure | 15850 | rot |
| 1-(2-Sulfo-4-methyl-5-chlor-1-phenylazo)-2-hydroxy-naphthalin-3-carbonsäure | 15865 | rot |
| 1-(2-Sulfo-1-naphthylazo)-2-hydroxynaphthalin-3-carbonsäure | 15880 | rot |
| 1-(3-Sulfo-1-phenylazo)-2-naphthol-6-sulfosäure | 15980 | orange |
| 1-(4-Sulfo-1-phenylazo)-2-naphthol-6-sulfosäure | 15985 | gelb |
| Allura Red | 16035 | rot |
| 1-(4-Sulfo-1-naphthylazo)-2-naphthol-3,6-disulfosäure | 16185 | rot |
| Acid Orange 10 | 16230 | orange |
| 1-(4-Sulfo-1-naphthylazo)-2-naphthol-6,8-disulfosäure | 16255 | rot |
| 1-(4-Sulfo-1-naphthylazo)-2-naphthol-3,6,8,-trisulfosäure | 16290 | rot |
| 8-Amino-2-phenylazo-1-naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4-Sulfo-1-phenylazo)-1-(4-sulfophenyl)-5-hydroxy-phrazolon-3-carbonsäure | 19140 | gelb |
| Pigment Yellow 16 | 20040 | gelb |
| 2,6-(4'-Sulfo-2",4"-dimethyl)-bis-phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"Sulfo-1"-phenylazo)-7'-sulfo-1'-naphthylazo]-1-hydroxy-7-aminoaphthalin-3,6-disulfosäure | 27755 | schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | schwarz |
| Direct Orange 34, 39, 44, 46, 60 | 40215 | orange |
| Food Yellow | 40800 | orange |
| trans-ß-Apo-8'-Carotinaldehyd (C₃₀) | 40820 | orange |
| trans-Apo-8'-Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"-bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino-phenyl-(2-sulfophenyl)-methylen-(N-ethyl-N-p-sulfo-benzyl)-cyclohexadienimin | 42090 | blau |
| Acid Green 9 | 42100 | grün |
| Diethyl-di-sulfobenzyl-di-4-amino-2-chlor-di-2-methyl-fuchsonimonium | 42170 | grün |
| Basic Violet 14 | 42510 | violett |
| Basic Violet 2 | 42520 | violett |
| 2'-Methyl-4'-(N-ethyl-N-m-sulfobenzyl)-amino-4"-(N-diethyl)-amino-2-methyl-N-ethyl-N-m-sulfobenzyl-fuchsonimmonium | 42735 | blau |
| 4'-(N-Dimethyl)-amino-4"-(N-phenyl)-aminonaphtho-N-dimethyl-fuchsonimmonium | 44045 | blau |
| 2-Hydroxy-3,6-disulfo-4,4'-bis-dimethylaminonaphtofuchsinimmonium | 44090 | grün |
| Acid red | 45100 | rot |
| 3-(2'-Methylphenylamino)-6-(2'-methyl-4'-sulfophenylamino)-9-(2"-carboxyphenyl)-xantheniumsalz | 45190 | violett |
| Acid Red 50 | 45220 | rot |
| Phenyl-2-oxyfluoron-2-carbonsäure | 45350 | gelb |
| 4,5-Dibromfluorescein | 45370 | orange |
| 2,4,5,7-Tetrabromfluorescein | 45380 | rot |
| Solvent Dye | 45396 | orange |
| Acid Red 98 | 45405 | rot |
| 3',4',5',6'-Tetrachlor-2,4,5,7-tetrabromfluorescein | 45410 | rot |
| 4,5-Diiodfluorescein | 45425 | rot |
| 2,4,5,7-Tetraiodfluorescein | 45430 | rot |
| Chinophthalon | 47000 | gelb |
| Chinophthalon-disulfosäure | 47005 | gelb |
| Acid violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)-anthrachinon | 60725 | violett |
| Acid Violet 23 | 60730 | violett |
| 1,4-Di(4'-methyl-phenylamino)-anthrachinon | 61565 | grün |
| 1,4-Bis-(o-sulfo-p-toluidine)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfosäure | 73015 | blau |
| 4,4'-Dimethyl-6,6'-dichlorthioindigo | 73360 | rot |
| 5,5'-Dichlor-7,7'-dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| chlorierte Phthalocyanine | 74260 | grün |
| Natural Yellow 6,19; Natural Red 1 | 75100 | gelb |
| Bixin, Nor-Bixin | 75120 | orange |
| Lycopin | 75125 | gelb |
| trans-alpha, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy-3-methoxyphenyl)1,6-heptadien-3,5-dion | 75300 | gelb |
| Komplexsalz (Na,Al,Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwarz |
| Pigment Black 9 | 77267 | schwarz |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydroxide | 77489 | orange |
| Eisenoxide und -hydroxide | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂*7H₂O | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyriliumsalze, Anthocyanine | | rot |
| Aluminium-, Zink-, Magnesium-, und Calciumstearat | | weiß |
| Bromthymolblau | | blau |
| Bromkresolgrün | | grün |
| Acid Red 195 | | rot |

Ferner vorteilhaft sind öllösliche Naturfarbstoffe, wie z.B. Paprikaextrakte, β-Carotin und Cochenille.

Vorteilhaft eingesetzt werden auch Perlglanzpigmente, z. B. Fischsilber (Guanin/ Hypoxanthin-Mischkristalle aus Fischschuppen) und Perlmutt (vermahlene Muschelschalen), monokristalline Perlglanzpigmente wie z. B. Bismuthoxychlorid (BiOCl), Schicht-Substrat Pigmente, z. B. Glimmer/Metalloxid, silberweiße Perlglanzpigmente aus TiO₂, Interferenpigmente (TiO₂, unterschiedliche Schichtdicke), Farbglanzpigmente (Fe₂O₃) und Kombinationspigmente (TiO₂/Fe₂, TiO₂/Cr₂O₃, TiO₂/Berliner Blau, TiO₂/Carmin).

Unter Effektpigmenten sind im Rahmen der vorliegenden Erfindung Pigmente zu verstehen, die durch ihre Brechungseigenschaften besondere optische Effekte hervorrufen. Effektpigmente verleihen der behandelten Oberfläche (Haut, Haar, Schleimhaut) Glanz- oder Glittereffekte oder können durch diffuse Lichtstreuung Hautunebenheiten und Hautfältchen optisch kaschieren. Als besondere Ausführungsform der Effektpigmente sind Interferenzpigmente bevorzugt. Besonders geeignete Effektpigmente sind beispielsweise Glimmerpartikel, die mit mindestens einem Metalloxid beschichtet sind. Neben Glimmer, einem Schichtsilikat, sind auch Kieselgel und andere SiO₂-Modifikationen als Träger geeignet. Ein häufig zur Beschichtung verwendetes Metalloxid ist beispielsweise Titanoxid, dem gewünschtenfalls Eisenoxid beigemischt sein kann. Über die Größe und die Form (z. B. sphärisch, ellipsoid, abgeflacht, eben, uneben) der Pigmentpartikel sowie über die Dicke der Oxidbeschichtung können die Reflexionseigenschaften beeinflusst werden. Auch andere Metalloxide, z. B. Bismutoxychlorid (BiOCl), sowie die Oxide von beispielsweise Titan, insbesondere die TiO₂-Modifikationen Anatas und Rutil, Aluminium, Tantal, Niob, Zirkon und Hafnium. Auch mit Magnesiumfluorid (MgF₂) und Calciumfluorid (Flussspat, CaF₂) können Effektpigmente hergestellt werden.

Die Effekte lassen sich sowohl über die Partikelgröße als auch über die Partikelgrößenverteilung des Pigmentensembles steuern. Geeignete Partikelgrößenverteilungen reichen z. B. von 2 - 50 µm, 5 - 25 µm, 5 - 40 µm, 5 - 60 µm, 5 - 95 µm, 5-100 µm, 10 - 60 µm, 10 - 100 µm, 10 - 125 µm, 20 - 100 µm, 20 - 150 µm, sowie < 15 µm. Eine breitere Teilchengrößenverteilung z. B. von 20 - 150 µm, ruft glitzernde Effekte hervor, während eine engere Teilchengrößenverteilung von < 15 µm für eine gleichmäßige seidige Erscheinung sorgt.

Die erfindungsgemäßen Zusammensetzungen enthalten Effektpigmente vorzugsweise in Mengen von 0,1 bis 20,0 Gew.-%, besonders bevorzugt von 0,5 bis 10,0 Gew.-% und insbesondere bevorzugt von 1,0 bis 5,0 Gew.-%, jeweils bezogen auf das Gesamtgewicht der Zusammensetzung.

Als deodorierende Stoffe bevorzugt sind Allantoin und Bisabolol. Diese werden vorzugsweise in Mengen von 0,0001 bis 10,0 Gew.-% eingesetzt.

Als Duft- bzw. Parfümöle können einzelne Riechstoffverbindungen, z. B. die synthetischen Produkte vom Typ der Ester, Ether, Aldehyde, Ketone, Alkohole und Kohlenwasserstoffe verwendet werden. Riechstoffverbindungen vom Typ der Ester sind z. B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethyl-methylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8 bis 18 C-Atomen, Citral, Citronellal, Citronellyloxyacetaldehyd, Cycllamenaldehyd, Hydroxycitronellal, Lilial und Bourgeonal, zu den Ketonen z. B. die lonone, alpha-Isomethylionon und Methyl-cedrylketon, zu den Alkoholen Anethol, Citronellol, Eugenol, Geranion, Linalol, Phenylethylalkohol und Terpineol, zu den Kohlenwasserstoffen gehören hauptsächlich die Terpene und Balsame. Bevorzugt werden Mischungen verschiedener Riechstoffe verwendet, die gemeinsam eine ansprechende Duftnote erzeugen.

Parfümöle können auch natürliche Riechstoffgemische enthalten, wie sie aus pflanzlichen oder tierischen Quellen zugänglich sind, z.B. Pinien-, Citrus-, Jasmin-, Lilien-, Rosen-, oder Ylang-Ylang-Öl. Auch ätherische Öle geringerer Flüchtigkeit, die meist als Aromakomponenten verwendet werden, eignen sich als Parfümöle, z. B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl und Ladanumöl.

Als perlglanzgebende Komponente bevorzugt geeignet sind Fettsäuremonoalkanolamide, Fettsäuredialkanolamide, Monoester oder Diester von Alkylenglykolen, insbesondere Ethylenglykol und/oder Propylenglykol oder dessen Oligomere, mit höheren Fettsäuren, wie z. B. Palmitinsäure, Stearinsäure und Behensäure, Monoester oder Polyester von Glycerin mit Carbonsäuren, Fettsäuren und deren Metallsalze, Ketosulfone oder Gemische der genannten Verbindungen. Besonders bevorzugt sind Ethylenglykoldistearate und/oder Polyethylenglykol-distearate mit durchschnittlich 3 Glykoleinheiten.

Sofern die erfindungsgemäßen Zusammensetzungen perlglanzgebende Verbindungen enthalten, sind diese bevorzugt in einer Menge von 0,1 bis 15,0 Gew.-% und besonders bevorzugt in einer Menge von 1,0 bis 10,0 Gew.-% in den erfindungsgemäßen Zusammensetzungen enthalten.

Als Säuren oder Laugen zur pH-Wert Einstellung werden vorzugsweise Mineralsäuren, insbesondere HCl, anorganische Basen, insbesondere NaOH oder KOH, und organische Säuren, insbesondere Zitronensäure, verwendet.

Die nachfolgenden Beispiele und Anwendungen sollen die Erfindung näher erläutern, ohne sie jedoch darauf zu beschränken. Bei allen Prozentangaben handelt es sich um Gewichts-% (Gew.-%).

### Herstellbeispiele, Allgemeine Arbeitsvorschrift:

Bei der Herstellung der erfindungsgemäßen Phosphorsäureester werden Phosphorsäure (85 %ig), Polyol und Fettalkoholalkoxylat in einem bestimmten Molverhältnis eingesetzt. Hierzu werden alle Edukte in einer Rührapparatur mit Heizpilz, Auskreiser mit Kühler und Vakuumanschluss vorgelegt. Die Mischung wird auf 100 °C aufgeheizt, dreimal bis 100 mbar evakuiert und anschließend mit Stickstoff wieder belüftet. Nach weiteren 4 Stunden Inertisierung (Stickstoffeinleitung von 20 Liter/Stunde) bei 100 °C wird der Ansatz unter Stickstoffeinleitung auf 230°C aufgeheizt und verestert (Wasseraustrag). Die Reaktionszeiten betragen 24 bis 42 Stunden (ab 230 °C Veresterungstemperatur gerechnet), insbesondere 40 Stunden. Die Rest-Säurezahl liegt dann bei < 15 mg KOH/g. Nach beendeter Umsetzung wird das Produkt auf 80 °C gekühlt, in eine Schale ausgegossen und die erstarrte Schmelze zerkleinert.

### Beispiel 1

Nach der allgemeinen Herstellvorschrift wurde ein Ester aus 18.2 g Phosphorsäure, 179.6 g Pentaerythrit + 100 Mol Ethylenoxid und 482.6 g Ceteareth-25 (C_{16/18} Fettalkohol + 25 Mol Ethylenoxid, Genapol^{®} T 250) im Molverhältnis 4 : 1 : 8 hergestellt. Die Rest-Säurezahl betrug 5.7 mg KOH/g, entsprechend 86 % Umsatz. Es handelt sich um ein weißes Wachs mit einem Schmelzpunkt von ca. 40 °C.

### Beispiel 2

Nach der allgemeinen Arbeitsvorschrift wurde ein Ester aus 22.8 g Phosphorsäure, 224.5 g Pentaerythrit + 100 Mol Ethylenoxid und 294.8 g Ceteareth 11 (C_{16/18} Fettalkohol + 11 Mol Ethylenoxid, Genapol^{®} T 110) im Molverhältnis 4 : 1 : 8 hergestellt. Die Restsäurezahl betrug 12.1 mg KOH/g entsprechend 80 % Umsatz. Es handelt sich um ein weißes Wachs mit einem Schmelzpunkt von ca. 38 °C.

### Viskositätsmessungen in wässrigen Lösungen

Die Viskositäten der Phosphorsäureester der Beispiele 1 und 2 im Vergleich zu den Handelsprodukten Crothix (PEG-150 Pentaerythrityl Tetrastearat), Genapol^{®} DAT 100 (PEG-150 Polyglyceryl-2 Tristearate) und Rewopal^{®} PEG 6000 DS (PEG-150 Distearate) wurde gemessen (jeweils 6 Gew.-% Produkt in Wasser). Die Viskosität wurde bei 20 °C mit einem Brookfield Viskosimeter Typ RVT bei 20 Upm gemessen sofort nach Herstellung der wässrigen Lösungen ("Viskosität sofort") und nach 3 Monaten Lagerung bei Raumtemperatur (RT).

**Tabelle 1 - Viskositäten in wässrigen Lösungen**

| Produkt | Viskosität sofort | Klarheit | Viskosität nach 3 Monaten Lagerung bei RT |
|---|---|---|---|
| | [mPa · s] | | [mPa · s] |
| Beispiel 1 | 164000 | klar | 88000 |
| Beispiel 2 | 98000 | klar | 60500 |
| PEG-150 Pentaerythrityltetrastearat | 6650 | trüb | 990 |
| PEG-150 Polyglyceryl-2-tristearat | 65 | trüb | n. b. |
| PEG-150 Distearat | 3900 | trüb | n. b. |

| | | | |
|---|---|---|---|
| PEG: Polyethylenglykol; RT: Raumtemperatur; n. b.: nicht bestimmt | | | |

Die Ergebnisse der Tabelle 1 zeigen, dass die erfindungsgemäßen Phosphorsäureester nach Beispiel 1 und 2 gegenüber den Vergleichsbeispielen in Wasser klare Gele von hoher Viskosität ergeben. Zudem ist die Viskositätserniedrigung bei Lagerung deutlich niedriger als z. B. bei PEG-150 Pentaerythrityltetrastearat.

### Viskositätsmessungen in wässrig-tensidischen Lösungen

Die Viskositäten der Phosphorsäureester der Beispiele 1 und 2 im Vergleich zu den Handelsprodukten Crothix (PEG-150 Pentaerythrityl Tetrastearat), Genapol^{®} DAT 100 (PEG-150 Polyglyceryl-2 Tristearate) und Rewopal^{®} PEG 6000 DS (PEG-150 Distearate) wurde gemessen (jeweils 1 Gew.-% Produkt in einer wässrigen Lösung von Natrium Laurethethersulfat mit 2 Ethylenoxid-Einheiten (INCl: Sodium Laureth Sulfate) : Cocamidopropylbetain im Verhältnis 8 : 2 mit einem Aktivsubstanzgehalt an Sodium Laureth Sulfate/Cocamidopropylbetain von 15 Gew.-% in Wasser; pH 4 - 4,4). Die Viskosität wurde bei 20°C mit einem Brookfield Viskosimeter Typ RVT bei 20 Upm gemessen sofort nach Herstellung der wässrig-tensidischen Lösungen ("Viskosität sofort") und nach 4 Monaten Lagerung bei 50 °C.

**Tabelle 2 Viskositäten in wässrig-tensidischen Lösungen**

| Produkt | Viskosität sofort | Viskosität nach 4 Monaten Lagerung bei 50 °C |
|---|---|---|
| | [mPa · s] | [mPa · s] |
| Beispiel 1 | 40600 | 45000 |
| Beispiel 2 | 69000 | 73000 |
| PEG-150 Pentaerythrityltetrastearat | 20000 | 80 |
| PEG-150 Polyglyceryl-2-tristearat | 23100 | 110 |
| PEG-150 Distearat | 2750 | 45 |

| | | |
|---|---|---|
| PEG: Polyethylenglykol | | |

Die Ergebnisse der Tabelle 2 zeigen, dass die erfindungsgemäf3en Phosphorsäureester nach Beispiel 1 und 2 im Vergleich zu den Vergleichsbeispielen in Tensid Gele von höherer Viskosität ergeben, die bei Lagerung im Gegensatz zu den Vergleichsbeispielen keine Viskosität verlieren.

### Formulierungsbeispiele:

| Formulierungsbeispiel 1 (Facial Cleansing Foam) | | |
|---|---|---|
| A | Stearinsäure | 1.60 % |
| | Myristinsäure | 1.80 % |
| | Laurinsäure | 0.70 % |
| | Tegin M | 0.50 % |
| | Glyceryl Stearate | |
| | Palmitinsäure | 0.70 % |
| | | |
| B | Wasser | ad 100.00 % |
| | | |
| C | Kaliumhydroxid | 0.70 % |
| | Phosphorsäureester Beispiel 1 | 1.10 % |

### Herstellung:

I A bei 80 °C aufschmelzen.
II C in B unter Rühren und bei 60 °C lösen, dann zu I geben.
III Unter Rühren abkühlen.

| Formulierungsbeispiel 2 (Cream Rinse) | | | |
|---|---|---|---|
| A | Genamin^{®} CTAC | (Clariant) | 6.00 % |
| | Cetrimonium Chloride | | |
| | Hostacerin^{®} DGL | (Clariant) | 1.50 % |
| | PEG-10 Diglyceryl-2 Laurate | | |
| | Cetylstearyl Alkohol | | 1.70 % |
| | Paraffinöl | | 1.00% |
| | | | |
| B | Wasser | | ad 100.00 % |
| C | Phosphorsäureester Beispiel 2 | | 0.80 % |
| | | | |
| D | Parfüm | | 0.30 % |
| | Panthenol | | 0.30 % |
| | Konservierungsmittel | | q.s. |
| | Farbstoff | | q.s. |

### Herstellung:

I A bei 75 °C aufschmelzen.
II C in B unter Rühren bei 60 °C lösen.
III II unter Rühren zu I geben. Kalt rühren.
IV Bei 40 °C die Komponenten von D zugeben.
V Den pH-Wert auf 4 einstellen.

| Formulierungsbeispiel 3 (Light Leave on for Hair tips) | | | |
|---|---|---|---|
| A | SilCare^{®} Silicone 41 M15 | (Clariant) | 0.30 % |
| | Caprylyl Methicone | | |
| | | | |
| B | Genapol^{®} LA 070 | (Clariant) | 8.00 % |
| | Laureth-7 | | |
| | | | |
| C | Wasser | | ad 100 % |
| | | | |
| D | Phosphorsäureester Beispiel 2 | | 1.50 % |
| | | | |
| E | Biobranil | | 0.50 % |
| | Soybean (Glycine Soja) Oil and Wheat | | |
| | (Triticum Vulgare) Bran Lipids | | |
| | Glycerin | | 2.00 % |
| | Panthenol | | 0.50 % |
| F | SilCare^{®} Silicone SEA | (Clariant) | 0.50 % |
| | Trideceth-9 PG Amodimethicone and Trideceth-12 | | |
| | Genamin^{®} CTAC | (Clariant) | 2.00 % |
| | Cetrimonium Chloride | | |
| | Nipaguard^{®} DMDMH | (Clariant) | 0.20 % |
| | DMDMH Hydantoin | | |

### Herstellung:

I A in B solubilisieren.
II D in C unter Rühren bei 60 °C lösen.
III E zu II geben und rühren bis die Lösung klar ist, danach zu I geben.
IV F zu III geben.

| Formulierungsbeispiel 4 (Wasserstoffperoxid Gel) | | | |
|---|---|---|---|
| A | Phosphorsäureester Beispiel 1 | | 3.00 % |
| | Genapol^{®} T 250 | (Clariant) | 2.00 % |
| | Ceteareth-25 | | |
| | | | |
| B | Wasser | | ad 100.00 % |
| | | | |
| C | Phosphorsäure | | 0.04 % |
| | Natriumdihydrogenphosphat | | 1.00 % |
| | | | |
| D | Wasserstoffperoxid 30 % ig | | 18.00 % |

### Herstellung:

I A unter Rühren und Erwärmen auf 50 °C in B lösen.
II Bei 25°C C zugeben.
III Bei Raumtemperatur D zugeben.

| Formulierungsbeispiel 5 (Deodorant Gel) | | | |
|---|---|---|---|
| A | Octopirox^{®} | (Clariant) | 0.10 % |
| | Piroctone Olamine | | |
| | | | |
| B | Emulsogen^{®} HCP 049 | (Clariant) | 10.00% |
| | PEG-40 Hydrogenated Castor Oil and Propylene Glycol | | |
| | Parfüm | | 0.20 % |
| | | | |
| C | Wasser | | ad 100.00 % |
| | | | |
| D | Phosphorsäureester Beispiel 2 | | 2.00 % |
| | | | |
| E | Citronensäure | | q.s. |

### Herstellung:

I A in B lösen.
II D in C unter Rühren und leichtem Erhitzen lösen, dann II zu ! geben.
III Wenn nötig den pH-Wert mit E auf 6.0 einstellen.

| Formulierungsbeispiel 6 (Make-Up Remover) | | | |
|---|---|---|---|
| A | Velsan^{®} P8-3 | (Clariant) | 5.00 % |
| | Isopropyl C12-15 Pareth-9 Carboxylate | | |
| | | | |
| B | Hostapon^{®} CGN | (Clariant) | 2.00 % |
| | Sodium Cocoyl Glutamate | | |
| | Genagen^{®} CAB | (Clariant) | 3.00 % |
| | Cocamidopropyl Betaine | | |
| | Allantoin | (Clariant) | 0.30 % |
| | Aristoflex^{®} PEA | (Clariant) | 1.00 % |
| | Polypropylene Terephthalate | | |
| | 1.6 Hexanediol | | 2.00 % |
| | 1.2 Propanediol | | 2.00 % |
| | Polyglykol 400 | (Clariant) | 2.00 % |
| | PEG-8 | | |
| | Panthenol | | 0.50 % |
| | Lutrol F127 | | 3.00 % |
| | Poloxamer 407 | | |
| | Konservierungsmittel | | q.s. |
| | | | |
| C | Phosphorsäureester Beispiel 1 | | 0.60 % |
| | | | |
| D | Wasser | | ad 100.00 % |
| | | | |
| E | Genapol^{®} LA 070 | (Clariant) | 2.00 % |
| | Laureth-7 | | |

### Herstellung:

I Nach und nach die Komponenten von B zu A geben und Rühren, bis eine klare Lösung entsteht.
II C in D unter Rühren und leichtem Erhitzen lösen, II zu I geben.
III E in I einrühren.

| Formulierungsbeispiel 7 (Whitening Gel) | | | |
|---|---|---|---|
| A | Wasser | | ad 100.00 % |
| | Arginin | | 1.10 % |
| | Phosphorsäureester Beispiel 1 | | 3.50 % |
| | | | |
| B | Dipropylenglykol | | 8.00% |
| | Genapol^{®}C 100 | (Clariant) | 0.60 % |
| | Coceth-10 | | |
| | Natrium Citrat*2H₂O | | 0.09% |
| | Citronensäure 10.0 % | | 0.10 % |
| | Nipagin^{®} M | (Clariant) | 0.20 % |
| | Methylparaben Ascorbinsäure-2-glucosid | | 2.00 % |

### Herstellung:

I Die Komponenten von A mischen und unter Rühren und leichtem Erhitzen lösen.
II Die Komponenten von B zu I geben und lösen. Falls nötig die Formulierung leicht erhitzen.

| Formulierungsbeispiel 8 (Facial Toner) | | | |
|---|---|---|---|
| A | Glycerin | | 8.00 % |
| | Polyglykol 400 | (Clariant) | 5.00 % |
| | PEG-8 | | |
| | Panthenol | | 0.50 % |
| | Parfüm | | 0.20 % |
| | Alkohol | | 8.00 % |
| | Konservierungsmittel | | q.s. |
| | Allantoin | (Clariant) | 0.10 % |
| | Niacinamide | | 0.10 % |
| | Extrapon Hamamelis | | 1.00 % |
| | Water, Witch Hazel Distillate, SD Alcohol 39-C, Butylene Glycol | | |
| | | | |
| B | Wasser | | ad 100 % |
| | | | |
| C | Phophorsäureester Beispiel 1 | | 2.50 % |

### Herstellung:

I C in B unter Rühren und leichtem Erhitzen lösen.
II Die Komponenten von A zu I geben und Rühren bis die Formulierung homogen ist.

| | | | |
|---|---|---|---|
| Formulierungsbeispiel 9 (Hairshampoo) | | | |
| A | Genapol^{®} LRO flüssig | (Clariant) | 30.00 % |
| | Sodium Laureth Sulfate | | |
| | Hostapon^{®} CGN | (Clariant) | 5.00 % |
| | Sodium Cocoyl Glutamate | | |
| | Parfüm | | 0.30 % |
| | | | |
| B | Wasser | | ad 100.00 % |
| | | | |
| C | Phosphorsäureester Beispiel 2 | | 1.55% |
| | Konservierungsmittel | | q.s. |
| | Farbstoff | | q.s. |
| | Genagen^{®} CAB | (Clariant) | 8.00 % |
| | Cocamidopropyl Betaine | | |

### Herstellung:

I C in B unter Rühren und Erhitzen auf 50 °C lösen.
II Nach und nach die Komponenten von A in I einrühren.
III Wenn nötig pH-Wert einstellen.

| Formulierungsbeispiel 10 (Schaumbad) | | | |
|---|---|---|---|
| A | Genapol^{®} LRO flüssig | (Clariant) | 60.00 % |
| | Sodium Laureth Sulfate | | |
| | | | |
| B | Medialan^{®} LD | (Clariant) | 8.00 % |
| | Sodium Lauroyl Sarcosinate | | |
| | Parfüm | | 1.50% |
| | Velsan^{®} CG 070 | (Clariant) | 5.00 % |
| | PEG-7 Glyceryl Cocoate | | |
| | | | |
| C | Phosphorsäureester Beispiel 2 | | 1.20 % |
| | | | |
| D | Wasser | | ad 100 % |
| | | | |
| E | Farbstoff | | q.s. |
| | Konservierungsmittel | | q.s. |
| | Genagen^{®} CAB | (Clariant) | 6.00 % |
| | Cocamidopropyl Betaine | | |

### Herstellung:

I Nach und nach die Komponenten von B in A einrühren.
II C in D unter Rühren und Erhitzen auf 50 °C lösen.
III I zur II geben.
VI E in III Rühren.
V Wenn nötig pH-Wert einstellen.

| | | | |
|---|---|---|---|
| Formulierungsbeispiel 11 (O/W-Skinmilk) | | | |
| A | Hostacerin^{®} DGI | (Clariant) | 2.00 % |
| | Polyglyceryl-2 Sesquiisostearate | | |
| | Isopropylpalmitat | | 4.00 % |
| | Octyldodecanol | | 4.00 % |
| | Nipaguard^{®} PDU | (Clariant) | q.s. |
| | Propylene Glycol (and) Diazolidinyl Urea (and) Methylparaben | | |
| | (and) Propylparaben | | |
| | | | |
| B | Aristoflex^{®} AVC | (Clariant) | 1.20 % |
| | Ammonium Acryloyldimethyltaurate/VP | Copolymer | |
| C | Hostapon^{®} CGN | (Clariant) | 0.60 % |
| | Sodium Cocoyl Glutamate | | |
| | Wasser | | ad 100 % |
| | | | |
| D | Phosphorsäureester Beispiel 2 | | 1.30 % |
| E | Parfüm | | 0.40 % |

### Herstellung:

I D in C unter Rühren und Erhitzen auf 50 °C lösen.
II B zu A geben, danach I zugeben und gut verrühren.
III E zur II geben.
IV Zum Schluss die Formulierung homogenisieren.

| Formulierungsbeispiel 12 (Antiperspirant Roll-on) | | | |
|---|---|---|---|
| A | Phosphorsäureester Beispiel 2 | | 1.50 % |
| B | Wasser | | ad 100.00 % |
| | | | |
| C | Locron^{®} L | (Clariant) | 20.00 % |
| | Aluminum Chlorohydrate | | |
| | | | |
| D | Genapol^{®} T 250 | (Clariant) | 5.00 % |
| | Ceteareth-25 | | |
| | Butylen Glykol | | 3.00 % |
| | Cetiol OE | | 1.00 % |
| | Dicaprylyl Ether | | |
| | Glyceryl Isostearat | | 2.00 % |
| E | SilCare^{®} Silicone SEA | (Clariant) | 0.50 % |
| | Trideceth-9 PG Amodimethicone and Trideceth-12 | | |

### Herstellung:

I A in B unter Rühren und Erhitzen auf 60 °C lösen.
II C zu I geben.
III D bei 50°C schmelzen und II zugeben, solange rühren, bis eine klare Lösung entstanden ist.
IV Bei 30 °C E zugeben.

Formulierungsbeispiel 12 zeigte gegenüber der gleichen Formulierung, aber ohne Phosphorsäureester nach Beispiel 2, eine deutliche Reduktion weißer Rückstände auf der Kleidung nach Anwendung des Roll-ons auf der Haut.

| | | | |
|---|---|---|---|
| Formulierungsbeispiel 13 (Vitamin C Gel) | | | |
| A | Phosphorsäureester Beispiel 2 | | 1.30 % |
| | Genapol^{®} T 250 | (Clariant) | 2.00 % |
| | Ceteareth-25 | | |
| | | | |
| B | Wasser | | ad 100.00 % |
| | | | |
| C | Ascorbinsäure | | 3.00 % |
| | | | |
| D | Aristoflex^{®} AVC | (Clariant) | 0.80 % |
| | Ammonium Acryloyldimethyltaurate/ VP Copolymer | | |

### Herstellung:

I A in B lösen unter Rühren bei 50 °C.
II C in I einrühren bei Raumtemperatur.
III D zugeben und solange rühren bis ein homogenes Gel entstanden ist.

| Formulierungsbeispiel 14 (Duschbad) | | | |
|---|---|---|---|
| A | Phosphorsäureester Beispiel 2 | (Clariant) | 2.50 % |
| | Aristoflex^{®} PEA | (Clariant) | 2.00 % |
| | Polypropylene-Terephthalate | | |
| | | | |
| B | Wasser | | ad 100 % |
| | | | |
| C | Genapol^{®} LRO flüssig | (Clariant) | 30.00 % |
| | Sodium Laureth Sulfate | | |
| | Genapol^{®} LA 030 | (Clariant) | 1.50 % |
| | Laureth-3 | | |
| | Hostapon^{®} CLG | (Clariant) | 5.00 % |
| | Sodium Lauroyl Glutamate | | |
| | Genagen^{®} KB | (Clariant) | 6.00 % |
| | Coco Betaine | | |
| | Parfüm | | 0.30 % |
| | Farbstoff | | q.s. |
| | Konservierungsmittel | | q.s. |

### Herstellung:

I A in B bei 50°C lösen.
II Die Komponenten von C nacheinander in I einrühren.
III Wenn nötig den pH Wert einstellen.

| Formulierungsbeispiel 15 (Facial Anti-Ageing Cream Gel) | | | |
|---|---|---|---|
| A | Phosphorsäureester Beispiel 1 | (Clariant) | 1.00 % |
| | | | |
| B | Wasser | | ad 100.00 % |
| C | Paraffinöl | | 5.00 % |
| | SilCare^{®} Silicone 31 M50 | (Clariant) | 3.00 % |
| | Caprylyl Trimethicone | | |
| | | | |
| D | Aristoflex^{®} AVC | (Clariant) | 1.80 % |
| | Ammonium Acryloyldimethyltaurate/VP Copolymer | | |
| | | | |
| E | Glycolsäure 30 % * | | 6.00 % |
| | Phenonip^{®} | (Clariant) | 0.50 % |
| | Phenoxyethanol (and) Methylparaben (and) Ethylparaben (and) Butylparaben (and) Propylparaben (and) Isobutylparaben | | |
| | | | |
| F | Genapol^{®} LA 070 | (Clariant) | 2.00 % |
| | Laureth-7 | | |

| | | | |
|---|---|---|---|
| * neutralisiert mit NaOH auf pH 4. | | | |

### Herstellung:

I A in B unter Rühren bei 50 °C lösen.
II D in C einrühren.
III I in II einrühren und solange rühren bis ein homogenes Gel entstanden ist.
IV E in III zugeben.
V F in IV einrühren und solange rühren bis das Cremegel homogen ist.

| Formulierungsbeispiel 16 (O/W Selbstbräunungscreme) | | | |
|---|---|---|---|
| A | Hostaphat^{®} CC 100 | (Clariant) | 1.0 % |
| | Cetyl Phosphate | | |
| | Glyceryl Stearate | | 0.5% |
| | Cetearyl Alkohol | | 0.5% |
| | Paraffinöl | | 8.0 % |
| | Isopropylpalmitat | | 7.0 % |
| | SilCare^{®} Silicone 41 M15 | (Clariant) | 1.0 % |
| | Caprylyl Methicone | | |
| | | | |
| B | Aristoflex^{®} AVC | (Clariant) | 1.2 % |
| | Ammonium Acryloyldimethyltaurate/VP Copolymer | | |
| | | | |
| C | Wasser | | ad 100 % |
| | | | |
| D | Phosphorsäureester Beispiel 2 | (Clariant) | 1.0 % |
| | | | |
| E | Hostapon^{®} CLG | (Clariant) | 0.5% |
| | Sodium Lauroyl Glutamate | | |
| | Glycerin | | 5.0 % |
| | | | |
| F | Tocopheryl Acetate | | 1.0 % |
| | Fragrance | | 0.2 % |
| | Preservative | | q.s. |
| | | | |
| G | Dihydroxyacetone | | 5.0 % |
| | | | |
| H | Wasser | | 8.0 % |
| | | | |
| I | Natriumhydroxid (10 % in Wasser) | | q.s. |

### Herstellung:

I A bei 80 °C aufschmelzen.
II B in A einrühren.
III D in C bei 50 °C lösen, dann E zugeben.
IV III in II einrühren.
V Bei Raumtemperatur F zugeben.
VI G in H lösen und in V einrühren.
VII Wenn notwendig den pH Wert mit I auf 4-5 einstellen.

| Formulierungsbeispiel 17 (O/W Sonnenschutzmilch) | | | |
|---|---|---|---|
| A | Hostaphat^{®} CK 100 | (Clariant) | 2.00 % |
| | Potassium Cetyl Phosphate | | |
| | SilCare^{®} Silicone 41M15 | (Clariant) | 1.00% |
| | Caprylyl Methicone | | |
| | Stearinsäure | | 0.50 % |
| | Cetyl Alkohol | | 0.50 % |
| | Cutina^{®} GMS | | 1.00 % |
| | Glyceryl Stearate | | |
| | Cetiol^{®} SN | | 4.00 % |
| | Cetearyl Isononanoat | | |
| | Velsan^{®} CCT | (Clariant) | 4.00 % |
| | Caprylic/Capric Triglyceride | | |
| | Neo^{®} Heliopan BB | | 1.50 % |
| | Benzophenone- 3 | | |
| | Eusolex^{®} 6300 | | 4.00% |
| | 4-Methylbenzylidene Camphor | | |
| | | | |
| B | Aristoflex^{®} AVC | (Clariant) | 0.40 % |
| | Ammonium Acryloyldimethyltaurate/VP Copolymer | | |
| | | | |
| C | Wasser | | ad 100 % |
| | | | |
| D | Phosphorsäureester Beispiel 2 | (Clariant) | 0.80 % |
| | | | |
| E | Glycerin | | 3.00 % |
| | Eusolex^{®} 232 | | 2.00 % |
| | Phenylbenzimidazole Sulfonic Acid | | |
| | Tris (hydroxymethyl)aminomethan | | 1.10 % |
| | Tromethamine | | |
| F | Tocopheryl Acetate | | 0.50 % |
| | Phenonip^{®} | (Clariant) | 0.50 % |
| | Phenoxyethanol (and) Methylparaben (and) Butylparaben (and) Ethylparaben (and) Propylparaben | | |
| | Fragrance | | 0.40 % |

### Herstellung:

I A bei 80 °C aufschmelzen, dann B hinzufügen.
II D in C bei 60°C lösen.
III E in II einrühren.
IV III in I lösen.
V Bei 35 °C F in IV zugeben.

| Formulierungsbeispiel 18 (Facial Anti-Ageing Gel) | | | |
|---|---|---|---|
| A | Genapol^{®} T 250 | (Clariant) | 1.00 % |
| | Ceteareth-25 | | |
| | Phosphorsäureester Beispiel 2 | (Clariant) | 1.40 % |
| | | | |
| B | Wasser | | ad 100 % |
| | | | |
| C | Aristoflex^{®} AVC | (Clariant) | 2.00 % |
| | Ammonium Acryloyldimethyltaurate/VP Copolymer | | |
| | | | |
| D | Glycolsäure 30%* | | 6.00 % |
| | Phenonip^{®} | (Clariant) | 0.50 % |
| | Phenoxyethanol (and) Methylparaben (and) Ethylparaben (and) Butylparaben (and) Propylparaben (and) Isobutylparaben | | |

| | | | |
|---|---|---|---|
| * neutralisiert mit NaOH auf pH 4. | | | |

### Herstellung:

I A in B unter Rühren bei 50 °C lösen.
II C zugeben und solange rühren bis ein homogenes Gel entstanden ist.
III D zugeben und rühren bis das Gel wieder homogen ist.

## Patentansprüche

1. Phosphorsäureester enthaltend
A) eine oder mehrere Struktureinheiten abgeleitet von Substanzen der Komponente a), wobei die Substanzen der Komponente a) ausgewählt sind aus Orthophosphorsäure und einem oder mehreren ihrer Derivate, und wobei das eine oder die mehreren Derivate der Orthophosphorsäure vorzugsweise ausgewählt sind aus Polyphosphorsäure, Tetraphosphordecaoxid, Phosphoroxychlorid und Phosphorpentachlorid,
B) eine oder mehrere Struktureinheiten abgeleitet von Substanzen der Komponente b), wobei die Substanzen der Komponente b) ausgewählt sind aus einer oder mehreren Verbindungen der Formel (I)
R2-O-(CH₂CH₂O)ᵤ(C₃H₆O)ᵥ(DO)_{w}-H (I)
worin
R² für eine lineare oder verzweigte, gesättigte Alkylgruppe mit 6 bis 30, vorzugsweise 8 bis 22 und besonders bevorzugt 12 bis 18 Kohlenstoffatomen, oder für eine lineare oder verzweigte, ein- oder mehrfach ungesättigte Alkenylgruppe mit 6 bis 30, vorzugsweise 8 bis 22 und besonders bevorzugt 12 bis 18 Kohlenstoffatomen, steht,
D für eine lineare oder verzweigte gesättigte Alkylengruppe mit 4 bis 20 Kohlenstoffatomen, für eine lineare oder verzweigte ein- oder mehrfach ungesättigte Alkenylengruppe mit 4 bis 20 Kohlenstoffatomen oder für -CH(Phenyl)CH₂- steht,
u für eine Zahl von 0 bis 200, vorzugsweise von 2 bis 150, besonders bevorzugt von 5 bis 100 und insbesondere bevorzugt von 10 bis 50, steht, v für eine Zahl von 0 bis 100, vorzugsweise von 0 bis 50 und besonders bevorzugt von 0 bis 20, steht,
w für eine Zahl von 0 bis 100, vorzugsweise von 0 bis 50 und besonders bevorzugt von 0 bis 20, steht, und
wobei die Gruppen CH₂CH₂O, C₃H₆O und DO aus den Verbindungen der Formel (I) blockartig oder statistisch verteilt angeordnet sein können, und
C) eine oder mehrere Struktureinheiten abgeleitet von Substanzen der Komponente c), wobei die Substanzen der Komponente c) ausgewählt sind aus einem oder mehreren Polyolen mit mehr als 2 OH-Gruppen, welche auch eine oder mehrere Alkoxylatgruppen tragen können und wobei die Alkoxylatgruppen jeweils aufgebaut sind aus einer oder mehreren Einheiten ausgewählt aus CH₂CH₂O-, C₃H₆O- und C₄H₈O-Einheiten, die innerhalb der Alkoxylatgruppen jeweils blockartig oder statistisch verteilt angeordnet sein können,
und wobei die Phosphorsäureester mindestens 2 über eine Struktureinheit abgeleitet von den Polyolen mit mehr als 2 OH-Gruppen oder abgeleitet von den Polyolen mit mehr als 2 OH-Gruppen, die ein oder mehrere der Alkoxylatgruppen tragen, verbrückte Phosphoratome pro Molekül enthalten.

2. Phosphorsäureester nach Anspruch 1, **dadurch gekennzeichnet, dass** die Substanzen der Komponente c) ausgewählt sind aus Glycerin, Diglycerin, Polyglycerin, Pentaerythrit, Dipentaerythrit, Pentaerythritoligomeren, Trimethylolpropan, Threit, Erythrit, Adonit, Arabit, Xylit, Mannit, Sorbitol, Inosit, Glucose, Mannose, Fructose, Sorbose, Arabinose, Xylose, Ribose, Mannopyranose, Galactopyranose, Glucopyranose, Maltose, Saccharose, Aminozucker, Ascorbinsäure, Glucamiden und Gluconamiden, welche auch eine oder mehrere Alkoxylatgruppen tragen können und wobei die Alkoxylatgruppen jeweils aufgebaut sind aus einer oder mehreren Einheiten ausgewählt aus CH₂CH₂O-, C₃H₆O- und C₄H₈O-Einheiten, die innerhalb der Alkoxylatgruppen jeweils blockartig oder statistisch verteilt angeordnet sein können.

3. Phosphorsäureester nach Anspruch 1 oder 2, **dadurch gekennzeichnet, dass** die Substanzen der Komponente c) ausgewählt sind aus Pentaerythrit, Glycerin und Diglycerin, vorzugsweise Pentaerythrit, welche auch eine oder mehrere Alkoxylatgruppen tragen können und wobei die Alkoxylatgruppen jeweils aufgebaut sind aus einer oder mehreren Einheiten ausgewählt aus CH₂CH₂O-, C₃H₆O- und C₄H₈O-Einheiten, die innerhalb der Alkoxylatgruppen jeweils blockartig oder statistisch verteilt angeordnet sein können.

4. Phosphorsäureester nach einem oder mehreren der Ansprüche 1 bis 3, **dadurch gekennzeichnet, dass** die Substanzen der Komponente c) eine oder mehrere Alkoxylatgruppen tragen.

5. Phosphorsäureester nach Anspruch 4, **dadurch gekennzeichnet, dass** die Alkoxylatgruppen der Substanzen der Komponente c) aus CH₂CH₂O-Gruppen bestehen und die Anzahl der CH₂CH₂O-Gruppen pro Polyol-Molekül mit mehr als 2 OH-Gruppen von 1 bis 150, bevorzugt von 5 bis 130 und besonders bevorzugt von 10 bis 110 beträgt.

6. Phosphorsäureester nach einem oder mehreren der Ansprüche 1 bis 4, **dadurch gekennzeichnet, dass** die Substanzen der Komponente b) ausgewählt sind aus einer oder mehreren Verbindungen der Formel (II),
R₂-O-(CH₂CH₂O)ᵤ(C₃H₆O)ᵥ₁-H (II)
worin
R² für eine lineare oder verzweigte, gesättigte Alkylgruppe mit 6 bis 30, vorzugsweise 8 bis 22 und besonders bevorzugt 12 bis 18 Kohlenstoffatomen, oder für eine lineare oder verzweigte, ein- oder mehrfach ungesättigte Alkenylgruppe mit 6 bis 30, vorzugsweise 8 bis 22 und besonders bevorzugt 12 bis 18 Kohlenstoffatomen, steht,
u1 für eine Zahl von 1 bis 200, vorzugsweise von 2 bis 150, besonders bevorzugt von 5 bis 100 und insbesondere bevorzugt von 10 bis 50, steht, und
v1 für eine Zahl von 1 bis 100, vorzugsweise von 1 bis 50 und besonders bevorzugt von 1 bis 20, steht,
und wobei die CH₂CH₂O- und C₃H₆O- Einheiten blockartig oder statistisch verteilt angeordnet sein können.

7. Phosphorsäureester nach einem oder mehreren der Ansprüche 1 bis 5, **dadurch gekennzeichnet, dass** die Substanzen der Komponente b) ausgewählt sind aus einer oder mehreren Verbindungen der Formel (III),
R₂-O-(CH₂CH₂O)ᵤ₁-H (III)
worin
R² für eine lineare oder verzweigte, gesättigte Alkylgruppe mit 6 bis 30, vorzugsweise 8 bis 22 und besonders bevorzugt 12 bis 18 Kohlenstoffatomen, oder für eine lineare oder verzweigte, ein- oder mehrfach ungesättigte Alkenylgruppe mit 6 bis 30, vorzugsweise 8 bis 22 und besonders bevorzugt 12 bis 18 Kohlenstoffatomen, steht, und
u1 für eine Zahl von 1 bis 200, vorzugsweise von 2 bis 150, besonders bevorzugt von 5 bis 100 und insbesondere bevorzugt von 10 bis 50, steht.

8. Phosphorsäureester nach einem oder mehreren der Ansprüche 1 bis 5 und 7, **dadurch gekennzeichnet, dass** die eine oder die mehreren Struktureinheiten abgeleitet von der einen oder den mehreren Verbindungen der Formel (I) solche Struktureinheiten sind, worin u für eine Zahl von 1 bis 200, vorzugsweise von 2 bis 150, besonders bevorzugt von 5 bis 100 und insbesondere bevorzugt von 10 bis 50, steht, v und w 0 sind, und der Rest R²-O- abgeleitet ist von Alkoholen ausgewählt aus Octanol, Decanol, Dodecanol, Tetradecanol, Hexadecanol, Octadecanol, Eicosanol, Behenylalkohol, Fettalkoholen mit C-Ketten-Schnitten zwischen 8 und 22, vorzugsweise C₁₀/C₁₂-Fettalkohol, C₁₂/C₁₄-Fettalkohol, C₁₂/C₁₅- Fettalkohol und C₁₆/C₁₈-Fettalkohol, verzweigten Fettalkoholen, vorzugsweise Guerbetalkoholen, und einfach ungesättigten Fettalkoholen, vorzugsweise Delta-9-cis-Hexadecanol, Delta-9-cis-Octadecanol, trans-9-Octadecanol und cis-Delta-11-Octadecanol.

9. Phosphorsäureester nach einem oder mehreren der Ansprüche 1 bis 5, 7 und 8, **dadurch gekennzeichnet, dass** die eine oder die mehreren Struktureinheiten abgeleitet von der einen oder den mehreren Verbindungen der Formel (I) Struktureinheiten sind abgeleitet von C₁₆/₁₈-Fettalkoholethoxylaten mit 10-50 Ethylenoxid-Einheiten, vorzugsweise abgeleitet von Substanzen ausgewählt aus C_{16/18}-Fettatkoholethoxylat mit 11 Ethylenoxid-Einheiten, C_{16/18}-Fettalkoholethoxylat mit 25 Ethylenoxid-Einheiten und C_{16/18}-Fettalkoholethoxylat mit 50 Ethylenoxid-Einheiten.

10. Phosphorsäureester nach einem oder mehreren der Ansprüche 1 bis 9, **dadurch gekennzeichnet, dass** die Gesamtzahl der in den Phosphorsäureestern enthaltenen Ethylenoxideinheiten in den Struktureinheiten abgeleitet von den Substanzen der Komponente b) und den Substanzen der Komponente c) zusammen pro Fettalkoholendgruppe hervorgegangen aus den Verbindungen der Formel (I) von 30 bis 100 und vorzugsweise von 40 bis 80, ist.

11. Phosphorsäureester nach einem oder mehreren der Ansprüche 1 bis 5 und 7 bis 10, **dadurch gekennzeichnet, dass** er erhältlich ist aus der Umsetzung von
a) 5 bis 10 Mol eines C₁₂-C₂₂-Fettalkoholethoxylats, vorzugsweise eines C_{16/18}-Fettalkoholethoxylats, mit 10-50 Ethylenoxid-Einheiten und vorzugsweise mit 11 oder 25 Ethylenoxid-Einheiten,
b) 1 Mol eines Polyols ausgewählt aus Pentaerythrit, Glycerin und Diglycerin jeweils ethoxyliert mit 50 bis 150 Ethylenoxideinheiten und
c) 2 bis 5 Mol Orthophosphorsäure oder einem oder mehreren ihrer Derivate, wobei das eine oder die mehreren Derivate der Orthophosphorsäure vorzugsweise ausgewählt sind aus Polyphosphorsäure, Tetraphosphordecaoxid, Phosphoroxychlorid und Phosphorpentachlorid.

12. Phosphorsäureester nach einem oder mehreren der Ansprüche 1 bis 5 und 7 bis 11, **dadurch gekennzeichnet, dass** er erhältlich ist aus der Umsetzung von
a) 6 bis 10 Mol, vorzugsweise 8 Mol, eines C₁₂-C₂₂-Fettalkoholethoxylats, vorzugsweise eines C_{16/18}- Fettalkoholethoxylats, mit 10-50 Ethylenoxid-Einheiten und vorzugsweise mit 11 oder 25 Ethylenoxid-Einheiten,
b) 1 Mol Pentaerythrit, ethoxyliert mit 50 bis 150, vorzugsweise 100, Ethylenoxideinheiten, und
c) 3 bis 5 Mol, vorzugsweise 4 Mol, Orthophosphorsäure oder einem oder mehreren ihrer Derivate, wobei das eine oder die mehreren Derivate der Orthophosphorsäure vorzugsweise ausgewählt sind aus Polyphosphorsäure, Tetraphosphordecaoxid, Phosphoroxychlorid und Phosphorpentachlorid.

13. Phosphorsäureester nach einem oder mehreren der Ansprüche 1 bis 12, **dadurch gekennzeichnet, dass** mindestens 75 %, vorzugsweise von 80 bis 100 %, besonders bevorzugt von 85 bis 100 % der aus den Substanzen der Komponente a) theoretisch maximal erhältlichen veresterbaren Funktionen in den Phosphorsäureestern verestert sind.

14. Verfahren zur Herstellung eines Phosphorsäureesters nach einem oder mehreren der Ansprüche 1 bis 13, **dadurch gekennzeichnet, dass** eine Phosphorsäurekomponente, ausgewählt aus Orthophosphorsäure und einem ihrer Derivate mit einer Alkoholkomponente, vorzugsweise Fettalkoholethoxylat, und Polyol mit mehr als 2 OH-Gruppen oder einem entsprechenden Alkoxylatgruppen-haltigen Polyol bei Temperaturen von 150 bis 250°C, bevorzugt von 180 bis 240 °C und besonders bevorzugt von 200 bis 230 °C umgesetzt wird, vorzugsweise ohne Zusatz eines Katalysators.

15. Verfahren nach Anspruch 14, **dadurch gekennzeichnet, dass** als Phosphorsäurekomponente eine Substanz ausgewählt aus Orthophosphorsäure, Polyphosphorsäure und Tetraphosphordecaoxid, vorzugsweise Orthophosphorsäure, umgesetzt wird.

16. Kosmetische, pharmazeutische oder dermatologische Zusammensetzung, **dadurch gekennzeichnet, dass** sie einen oder mehrere Phosphorsäureester nach einem oder mehreren der Ansprüche 1 bis 13 enthält.

17. Zusammensetzung nach Anspruch 16, **dadurch gekennzeichnet, dass** sie in Form einer wässrigen, wässrig-alkoholischen oder wässrig-tensidischen Zusammensetzung, einer Emulsion, Suspension, Dispersion, eines Puders oder eines Sprays vorliegt.

18. Zusammensetzung nach Anspruch 16 oder 17, **dadurch gekennzeichnet, dass** sie einen pH-Wert von 2 bis 10, vorzugsweise von 2 bis 6, besonders bevorzugt von 2,5 bis 5 und insbesondere bevorzugt von 3 bis 4,5, besitzt.

19. Zusammensetzung nach einem oder mehreren der Ansprüche 16 bis 18, **dadurch gekennzeichnet, dass** sie einen oder mehrere Elektrolyte enthält.

20. Zusammensetzung nach Anspruch 19, **dadurch gekennzeichnet, dass** der Gehalt an dem einen oder den mehreren Elektrolyten, bezogen auf die gesamte Zusammensetzung, von 0,1 bis 20,0 Gew.-%, vorzugsweise von 0,2 bis 10,0 Gew.-% und besonders bevorzugt von 0,5 bis 5,0 Gew.-% ist.

21. Zusammensetzung nach einem oder mehreren der Ansprüche 16 bis 20, **dadurch gekennzeichnet, dass** sie Wasserstoffperoxid oder Wasserstoffperoxid-freisetzende Substanzen enthält, und vorzugsweise in der Form eines Gels oder einer Creme vorliegt.

22. Zusammensetzung nach einem oder mehreren der Ansprüche 16 bis 21, **dadurch gekennzeichnet, dass** sie ein oder mehrere Tenside enthält.

23. Zusammensetzung nach einem oder mehreren der Ansprüche 16 bis 22, **dadurch gekennzeichnet, dass** sie den einen oder die mehreren Phosphorsäureester nach einem oder mehreren der Ansprüche 1 bis 13, bezogen auf die fertige Zusammensetzung, in einer Menge von 0,01 bis 10,0 Gew.-%, bevorzugt von 0,1 bis 6,0 Gew.-% und besonders bevorzugt von 0,5 bis 3,0 Gew.-% enthält.

24. Verwendung eines oder mehrerer der Phosphorsäureester nach einem oder mehreren der Ansprüche 1 bis 13 als Verdicker, Konsistenzgeber, Emulgator, Sensorikadditiv, Solubilisator, Dispergator, Gleitmittel, Haftmittel oder Stabilisator, vorzugsweise als Verdicker.

25. Verwendung eines oder mehrerer Phosphorsäureester nach einem oder mehreren der Ansprüche 1 bis 13 in Deodorant-oder Antiperspirant-Formulierungen, insbesondere in Deodorant- oder Antiperspirant-Formulierungen enthaltend Aluminiumsalze, bevorzugt Aluminiumchlorohydrat oder Aluminium-Zirconium-Komplexsalze, zur Verringerung der Bildung weißer Rückstände auf der Kleidung nach Anwendung der Deodorant- oder Antiperspirant-Formulierungen auf der Haut.

## Claims

1. A phosphoric ester comprising
A) one or more structural units derived from substances of component a), the substances of component a) being selected from orthophosphoric acid and one or more of its derivatives and the one or more derivatives of orthophosphoric acid preferably being selected from polyphosphoric acid, tetraphosphorus decaoxide, phosphoryl chloride and phosphorus pentachloride,
B) one or more structural units derived from substances of component b), the substances of component b) being selected from one or more compounds of formula (I)
R²-O-(CH₂CH₂O)ᵤ(C₃H₆O)ᵥ(DO)_{w}-H (I)
where
R² is a linear or branched, saturated alkyl group having 6 to 30, preferably 8 to 22 and more preferably 12 to 18 carbon atoms, or is a linear or branched mono- or polyunsaturated alkenyl group having 6 to 30, preferably 8 to 22 and more preferably 12 to 18 carbon atoms,
D is a linear or branched saturated alkylene group having 4 to 20 carbon atoms, is a linear or branched mono- or polyunsaturated alkenylene group having 4 to 20 carbon atoms or is -CH(phenyl)CH₂-,
u is a number from 0 to 200, preferably from 2 to 150, more preferably from 5 to 100 and even more preferably from 10 to 50,
v is a number from 0 to 100, preferably from 0 to 50 and more preferably from 0 to 20,
w is a number from 0 to 100, preferably from 0 to 50 and more preferably from 0 to 20, and
where the groups CH₂CH₂O, C₃H₆O and DO from the compounds of formula (I) can be arranged blocklike or randomly distributed, and
C) one or more structural units derived from substances of component c), the substances of component c) being selected from one or more polyols having more than 2 OH groups which may also bear one or more alkoxylate groups and where the alkoxylate groups are each constructed of one or more units selected from CH₂CH₂O-, C₃H₆O- and C₄H₈O- units which may each be arranged blocklike or randomly distributed within the alkoxylate groups,
and where the phosphoric esters contain at least 2 phosphorus atoms per molecule which are bridged via a structural unit derived from the polyols having more than 2 OH groups or derived from the polyols having more than 2 OH groups which bear one or more of the alkoxylate groups.

2. The phosphoric ester according to claim 1 wherein the substances of component c) are selected from glycerol, diglycerol, polyglycerol, pentaerythritol, dipentaerythritol, pentaerythritol oligomers, trimethylolpropane, threitol, erythritol, adonitol, arabitol, xylitol, mannitol, sorbitol, inositol, glucose, mannose, fructose, sorbose, arabinose, xylose, ribose, mannopyranose, galactopyranose, glucopyranose, maltose, sucrose, amino sugar, ascorbic acid, glucamides and gluconamides, which may also bear one or more alkoxylate groups and where the alkoxylate groups are each constructed of one or more units selected from CH₂CH₂O-, C₃H₆O- and C₄H₈O- units which each may be arranged blocklike or randomly distributed within the alkoxylate groups.

3. The phosphoric ester according to claim 1 or 2 wherein the substances of component c) are selected from pentaerythritol, glycerol and diglycerol, preferably pentaerythritol, which may also bear one or more alkoxylate groups and where the alkoxylate groups are each constructed of one or more units selected from CH₂CH₂O-, C₃H₆O- and C₄H₈O- units which each may be arranged blocklike or randomly distributed within the alkoxylate groups.

4. The phosphoric ester according to one or more of claims 1 to 3 wherein the substances of component c) bear one or more alkoxylate groups.

5. The phosphoric ester according to claim 4 wherein the alkoxylate groups of the substances of component c) consist of CH₂CH₂O-groups and the number of CH₂CH₂O-groups per polyol molecule having more than 2 OH groups is in the range from 1 to 150, preferably in the range from 5 to 130 and more preferably in the range from 10 to 110.

6. The phosphoric ester according to one or more of claims 1 to 4 wherein the substances of component b) are selected from one or more compounds of formula (II),
R²-O- (CH₂CH₂O)ᵤ₁(C₃H₆O)ᵥ₁-H (II)
where
R² is a linear or branched saturated alkyl group having 6 to 30, preferably 8 to 22 and more preferably 12 to 18 carbon atoms, or is a linear or branched mono- or polyunsaturated alkenyl group having 6 to 30, preferably 8 to 22 and more preferably 12 to 18 carbon atoms,
u1 is a number from 1 to 200, preferably from 2 to 150, more preferably from 5 to 100 and even more preferably from 10 to 50, and
v1 is a number from 1 to 100, preferably from 1 to 50 and more preferably from 1 to 20,
and wherein the CH₂CH₂O- and C₃H₆O- units may be arranged blocklike or randomly distributed.

7. The phosphoric ester according to one or more of claims 1 to 5 wherein the substances of component b) are selected from one or more compounds of formula (III),
R²-O-(CH₂CH₂O)ᵤ₁-H (III)
where
R² is a linear or branched saturated alkyl group having 6 to 30, preferably 8 to 22 and more preferably 12 to 18 carbon atoms, or is a linear or branched mono- or polyunsaturated alkenyl group having 6 to 30, preferably 8 to 22 and more preferably 12 to 18 carbon atoms, and
u1 is a number from 1 to 200, preferably from 2 to 150, more preferably from 5 to 100 and even more preferably from 10 to 50.

8. The phosphoric ester according to one or more of claims 1 to 5 and 7 wherein the one or more structural units derived from the one or more compounds of formula (I) are such structural units wherein u is a number from 1 to 200, preferably from 2 to 150, more preferably from 5 to 100 and even more preferably from 10 to 50, v and w are 0 and the radical R²-O- is derived from alcohols selected from octanol, decanol, dodecanol, tetradecanol, hexadecanol, octadecanol, eicosanol, behenyl alcohol, fatty alcohols having C-chain cuts between 8 and 22, preferably C₁₀/C₁₂ fatty alcohol, C₁₂/C₁₄ fatty alcohol, C₁₂/C₁₅ fatty alcohol and C₁₆/C₁₈ fatty alcohol, branched fatty alcohols, preferably Guerbet alcohols and monounsaturated fatty alcohols and preferably delta-9-cis-hexadecanol, delta-9-cis-octadecanol, trans-9-octadecanol and cis-delta-11-octadecanol.

9. The phosphoric ester according to one or more of claims 1 to 5, 7 and 8 wherein the one or more structural units derived from the one or more compounds of formula (I) are structural units derived from C_{16/18} fatty alcohol ethoxylates having 10-50 ethylene oxide units, preferably derived from substances selected from C_{16/18} fatty alcohol ethoxylate having 11 ethylene oxide units, C_{16/18} fatty alcohol ethoxylate having 25 ethylene oxide units and C_{16/18} fatty alcohol ethoxylate having 50 ethylene oxide units.

10. The phosphoric ester according to one or more of claims 1 to 9 wherein the total number in the phosphoric ester of ethylene oxide units in the structural units derived from the substances of component b) and the substances of component c) is together in the range from 30 to 100 and preferably in the range from 40 to 80 per fatty alcohol end group emerged from the compounds of formula (I).

11. The phosphoric ester according to one or more of claims 1 to 5 and 7 to 10 wherein it is obtainable from the reaction of
a) 5 to 10 mol of a C₁₂-C₂₂ fatty alcohol ethoxylate, preferably of a C_{16/18} fatty alcohol ethoxylate having 10-50 ethylene oxide units and preferably having 11 or 25 ethylene oxide units,
b) 1 mol of a polyol selected from pentaerythritol, glycerol and diglycerol each ethoxylated with 50 to 150 ethylene oxide units, and
c) 2 to 5 mol of orthophosphoric acid or one or more of its derivatives, in which case the one or more derivatives of orthophosphoric acid are preferably selected from polyphosphoric acid, tetraphosphorus decaoxide, phosphoryl chloride and phosphorus pentachloride.

12. The phosphoric ester according to one or more of claims 1 to 5 and 7 to 11 wherein it is obtainable from the reaction of
a) 6 to 10 mol, preferably 8 mol, of a C₁₂-C₂₂ fatty alcohol ethoxylate, preferably of a C_{16/18} fatty alcohol ethoxylate having 10-50 ethylene oxide units and preferably having 11 or 25 ethylene oxide units,
b) 1 mol of pentaerythritol ethoxylated with 50 to 150 and preferably 100 ethylene oxide units, and
c) 3 to 5 mol, preferably 4 mol, of orthophosphoric acid or one or more of its derivatives, in which case the one or more derivatives of orthophosphoric acid are preferably selected from polyphosphoric acid, tetraphosphorus decaoxide, phosphoryl chloride and phosphorus pentachloride.

13. The phosphoric ester according to one or more of claims 1 to 12 wherein at least 75%, preferably from 80 to 100% and more preferably from 85 to 100% of the maximum number of the esterifiable functions theoretically obtainable from the substances of component a) in the phosphoric ester are in an esterified state.

14. A process for preparing a phosphoric ester according to one or more of claims 1 to 13, which comprises reacting a phosphoric acid component selected from orthophosphoric acid and one of its derivatives with an alcohol component, preferably fatty alcohol ethoxylate, and polyol having more than 2 OH groups or a corresponding polyol comprising alkoxylate groups at temperatures of 150 to 250°C, preferably of 180 to 240°C and more preferably of 200 to 230°C, preferably without addition of a catalyst.

15. The process according to claim 14 wherein a substance selected from orthophosphoric acid, polyphosphoric acid and tetraphosphorus decaoxide, preferably orthophosphoric acid, is reacted as phosphoric acid component.

16. A cosmetic, pharmaceutical or dermatological composition, comprising one or more phosphoric esters according to one or more of claims 1 to 13.

17. The composition according to claim 16 in the form of an aqueous, aqueous-alcoholic or aqueous-surfactant composition, in the form of an emulsion, in the form of a suspension, in the form of a dispersion, in the form of a powder or in the form of a spray.

18. The composition according to claim 16 or 17 having a pH in the range from 2 to 10, preferably from 2 to 6, more preferably from 2.5 to 5 and even more preferably from 3 to 4.5.

19. The composition according to one or more of claims 16 to 18 comprising one or more electrolytes.

20. The composition according to claim 19 wherein the content of the one or more electrolytes is from 0.1% to 20.0% by weight, preferably 0.2% to 10.0% by weight and more preferably from 0.5% to 5.0% by weight, based on the entire composition.

21. The composition according to one or more of claims 16 to 20 comprising hydrogen peroxide or hydrogen peroxide releasers and preferably being present in the form of a gel or a cream.

22. The composition according to one or more of claims 16 to 21 comprising one or more surfactants.

23. The composition according to one or more of claims 16 to 22 comprising the one or more phosphoric esters according to one or more of claims 1 to 13 in an amount of 0.01% to 10.0% by weight, preferably 0.1% to 6.0% by weight and more preferably 0.5% to 3.0% by weight, based on the final composition.

24. The use of one or more of the phosphoric esters according to one or more of claims 1 to 13 as thickener, consistency regulator, emulsifier, sensory additive, solubilizer, dispersant, glidant, adhesive or stabilizer preferably as thickener.

25. The use of one or more phosphoric esters according to one or more of claims 1 to 13 in deodorant or antiperspirant formulations, in particular in deodorant or antiperspirant formulations comprising aluminum salts, preferably aluminum chlorohydrate or aluminum-zirconium complex salts, for reducing the formation of white residues on the clothing after using the deodorant or antiperspirant formulations on the skin.

## Revendications

1. Ester de l'acide phosphorique, contenant
A) une ou plusieurs unités de structure dérivées de substances du composant a), où les substances du composant a) sont choisies parmi l'acide orthophosphorique et un ou plusieurs de ses dérivés et où ledit un ou lesdits plusieurs dérivé(s) de l'acide orthophosphorique sont de préférence choisis parmi l'acide polyphosphorique, le décaoxyde de tétraphosphore, l'oxychlorure de phosphore et le pentachlorure de phosphore,
B) une ou plusieurs unités de structure dérivées de substances du composant b), où les substances du composant b) sont choisies parmi un ou plusieurs composés de formule (I)
R²-O-(CH₂CH₂O)ᵤ(C₃H₆O)ᵥ(DO)_{w}-H (I)
où
R² représente un groupe alkyle linéaire ou ramifié, saturé, comprenant 6 à 30, de préférence 8 à 22 et de manière particulièrement préférée 12 à 18 atomes de carbone, ou un groupe alcényle linéaire ou ramifié, monoinsaturé ou polyinsaturé, comprenant 6 à 30, de préférence 8 à 22 et de manière particulièrement préférée 12 à 18 atomes de carbone,
D représente un groupe alkylène linéaire ou ramifié, saturé, comprenant 4 à 20 atomes de carbone, un groupe alcénylène linéaire ou ramifié, monoinsaturé ou polyinsaturé, comprenant 4 à 20 atomes de carbone ou -CH(phényl)CH₂-,
u représente un nombre de 0 à 200, de préférence de 2 à 150, de manière particulièrement préférée de 5 à 100 et en particulier de préférence de 10 à 50,
v représente un nombre de 0 à 100, de préférence de 0 à 50 et de manière particulièrement préférée de 0 à 20,
w représente un nombre de 0 à 100, de préférence de 0 à 50 et de manière particulièrement préférée de 0 à 20, et
où les groupes CH₂CH₂O, C₃H₆O et DO des composés de formule (I) peuvent être disposés en étant répartis par blocs ou statistiquement, et
C) une ou plusieurs unités de structure dérivées de substances du composant c), où les substances du composant c) sont choisies parmi un ou plusieurs polyols comprenant plus de 2 groupes OH, qui peuvent également porter un ou plusieurs groupes alcoxylate et où les groupes alcoxylate sont à chaque fois construits à partir d'une ou de plusieurs unités choisies parmi les unités CH₂CH₂O, C₃H₆O et C₄H₈O, qui peuvent être disposées en étant réparties respectivement par blocs ou statistiquement dans les groupes alcoxylate,
et où les esters d'acide phosphorique contiennent, par molécule, au moins 2 atomes de phosphore pontés via une unité de structure dérivée des polyols comprenant plus de 2 groupes OH ou dérivée des polyols comprenant plus de 2 groupes OH qui portent un ou plusieurs groupes alcoxylate.

2. Ester de l'acide phosphorique selon la revendication 1, **caractérisé en ce que** les substances du composant c) sont choisies parmi le glycérol, le diglycérol, le polyglycérol, le pentaérythritol, le dipentaérythritol, les oligomères de pentaérythritol, le triméthylolpropane, le thréitol, l'érythritol, l'adonitol, l'arabitol, le xylitol, le mannitol, le sorbitol, l'inositol, le glucose, le mannose, le fructose, le sorbose, l'arabinose, le xylose, le ribose, le mannopyranose, le galactopyranose, le glucopyranose, le maltose, le saccharose, les aminosucres, l'acide ascorbique, les glucamides et les gluconamides, qui peuvent également porter un ou plusieurs groupes alcoxylate et où les groupes alcoxylate sont à chaque fois construits à partir d'une ou de plusieurs unités choisies parmi les unités CH₂CH₂O, C₃H₆O et C₄H₈O, qui peuvent être disposées en étant réparties respectivement par blocs ou statistiquement dans les groupes alcoxylate.

3. Ester de l'acide phosphorique selon la revendication 1 ou 2, **caractérisé en ce que** les substances du composant c) sont choisies parmi le pentaérythritol, le glycérol et le diglycérol, de préférence le pentaérythritol, lesquelles peuvent également porter un ou plusieurs groupes alcoxylate et où les groupes alcoxylate sont à chaque fois construits à partir d'une ou de plusieurs unités choisies parmi les unités CH₂CH₂O, C₃H₆O et C₄H₈O, qui peuvent être disposées en étant réparties respectivement par blocs ou statistiquement dans les groupes alcoxylate.

4. Ester de l'acide phosphorique selon l'une ou plusieurs des revendications 1 à 3, **caractérisé en ce que** les substances du composant c) portent un ou plusieurs groupes alcoxylate.

5. Ester de l'acide phosphorique selon la revendication 4, **caractérisé en ce que** les groupes alcoxylate des substances du composant c) sont constitués par des groupes CH₂CH₂O et le nombre de groupes CH₂CH₂O par molécule de polyol comprenant plus de 2 groupes OH est de 1 à 150, de préférence de 5 à 130 et de manière particulièrement préférée de 10 à 110.

6. Ester de l'acide phosphorique selon l'une ou plusieurs des revendications 1 à 4, **caractérisé en ce que** les substances du composant b) sont choisies parmi un ou plusieurs composés de formule (II)
R²-O-(CH₂CH₂O)ᵤ₁(C₃H₆O)ᵥ₁-H (II)
où
R² représente un groupe alkyle linéaire ou ramifié, saturé, comprenant 6 à 30, de préférence 8 à 22 et de manière particulièrement préférée 12 à 18 atomes de carbone, ou un groupe alcényle linéaire ou ramifié, monoinsaturé ou polyinsaturé, comprenant 6 à 30, de préférence 8 à 22 et de manière particulièrement préférée 12 à 18 atomes de carbone,
u1 représente un nombre de 1 à 200, de préférence de 2 à 150, de manière particulièrement préférée de 5 à 100 et en particulier de préférence de 10 à 50, et
v1 représente un nombre de 1 à 100, de préférence de 1 à 50, de manière particulièrement préférée de 1 à 20,
et où les groupes CH₂CH₂O et C₃H₆O peuvent être disposés en étant répartis par blocs ou statistiquement.

7. Ester de l'acide phosphorique selon l'une ou plusieurs des revendications 1 à 5, **caractérisé en ce que** les substances du composant b) sont choisies parmi un ou plusieurs composés de formule (III)
R²-O- (CH₂CH₂O)ᵤ₁-H (III)
où
R² représente un groupe alkyle linéaire ou ramifié, saturé, comprenant 6 à 30, de préférence 8 à 22 et de manière particulièrement préférée 12 à 18 atomes de carbone, ou un groupe alcényle linéaire ou ramifié, monoinsaturé ou polyinsaturé, comprenant 6 à 30, de préférence 8 à 22 et de manière particulièrement préférée 12 à 18 atomes de carbone, et
u1 représente un nombre de 1 à 200, de préférence de 2 à 150, de manière particulièrement préférée de 5 à 100 et en particulier de préférence de 10 à 50.

8. Ester de l'acide phosphorique selon l'une ou plusieurs des revendications 1 à 5 et 7, **caractérisé en ce que** ladite une ou lesdites plusieurs unités de structure dérivée(s) dudit un ou desdits plusieurs composé(s) de formule (I) sont les unités de structure dans lesquelles u représente un nombre de 1 à 200, de préférence de 2 à 150, de manière particulièrement préférée de 5 à 100 et en particulier de préférence de 10 à 50, v et w valent 0, et le radical R²-O- est dérivé d'alcools choisis parmi l'octanol, le décanol, le dodécanol, le tétradécanol, l'hexadécanol, l'octadécanol, l'eicosanol, l'alcool béhénylique, les alcools gras avec des fractions de chaînes C entre 8 et 22, de préférence l'alcool gras en C₁₀/C₁₂, l'alcool gras en C₁₂/C₁₄, l'alcool gras en C₁₂/C₁₅ et l'alcool gras en C₁₆/C₁₈, les alcools gras ramifiés, de préférence les alcools de Guerbet, et les alcools gras monoinsaturés, de préférence le delta-9-cis-hexadécanol, le delta-9-cis-octadécanol, le trans-9-octadécanol et le cis-delta-11-octadécanol.

9. Ester de l'acide phosphorique selon l'une ou plusieurs des revendications 1 à 5, 7 et 8, **caractérisé en ce que** ladite une ou lesdites plusieurs unités de structure dérivée(s) dudit un ou desdits plusieurs composé(s) de formule (I) sont des unités de structure dérivées d'éthoxylates d'alcool gras en C_{16/18} comprenant 10-50 unités d'oxyde d'éthylène, de préférence dérivées de substances choisies parmi l'éthoxylate d'alcool gras en C_{16/18} comprenant 11 unités d'oxyde d'éthylène, l'éthoxylate d'alcool gras en C_{16/18} comprenant 25 unités d'oxyde d'éthylène et l'éthoxylate d'alcool gras en C_{16/18} comprenant 50 unités d'oxyde d'éthylène.

10. Ester de l'acide phosphorique selon l'une ou plusieurs des revendications 1 à 9, **caractérisé en ce que** la totalité des unités d'oxyde d'éthylène contenues dans les esters de l'acide phosphorique dans les unités de structure dérivées des substances du composant b) et des substances du composant c) ensemble par groupe terminal d'alcool gras provenant des composés de formule (I) est de 30 à 100 et de préférence de 40 à 80.

11. Ester de l'acide phosphorique selon l'une ou plusieurs des revendications 1 à 5 et 7 à 10, **caractérisé en ce qu'**il peut être obtenu par la transformation de
a) 5 à 10 moles d'un éthoxylate d'alcool gras en C₁₂-C₂₂, de préférence d'un éthoxylate d'alcool gras en C_{16/18} présentant 10-50 unités d'oxyde d'éthylène et de préférence 11 ou 25 unités d'oxyde d'éthylène,
b) 1 mole d'un polyol choisi parmi le pentaérythritol, le glycérol et le diglycérol, à chaque fois éthoxylé avec 50 à 150 unités d'oxyde d'éthylène et
c) 2 à 5 moles d'acide orthophosphorique ou d'un ou de plusieurs de ses dérivés,
où ledit un ou lesdits plusieurs dérivé(s) de l'acide orthophosphorique sont de préférence choisis parmi l'acide polyphosphorique, le décaoxyde de tétraphosphore, l'oxychlorure de phosphore et le pentachlorure de phosphore.

12. Ester de l'acide phosphorique selon l'une ou plusieurs des revendications 1 à 5 et 7 à 11, **caractérisé en ce qu'**il peut être obtenu par la transformation de
a) 6 à 10 moles, de préférence 8 moles, d'un éthoxylate d'alcool gras en C₁₂-C₂₂, de préférence d'un éthoxylate d'alcool gras en C_{16/18} présentant 10-50 unités d'oxyde d'éthylène et de préférence 11 ou 25 unités d'oxyde d'éthylène,
b) 1 mole de pentaérythritol, éthoxylée avec 50 à 150, de préférence 100, unités d'oxyde d'éthylène, et
c) 3 à 5 moles, de préférence 4 moles, d'acide orthophosphorique ou d'un ou plusieurs de ses dérivés où ledit un ou lesdits plusieurs dérivé(s) de l'acide orthophosphorique sont de préférence choisis parmi l'acide polyphosphorique, le décaoxyde de tétraphosphore, l'oxychlorure de phosphore et le pentachlorure de phosphore.

13. Ester de l'acide phosphorique selon l'une ou plusieurs des revendications 1 à 12, **caractérisé en ce qu'**au moins 75%, de préférence 80 à 100%, de manière particulièrement préférée 85 à 100% des fonctions estérifiables pouvant être obtenues théoriquement au maximum à partir des substances du composant a) sont estérifiées dans les esters de l'acide phosphorique.

14. Procédé pour la préparation d'un ester de l'acide phosphorique selon l'une ou plusieurs des revendications 1 à 13, **caractérisé en ce que** l'on transforme un composant de l'acide phosphorique, choisi parmi l'acide orthophosphorique et un de ses dérivés avec un composant alcool, de préférence un éthoxylate d'alcool gras, et un polyol comprenant plus de 2 groupes OH ou un polyol contenant des groupes alcoxylate correspondant à des températures de 150 à 250°C, de préférence de 180 à 240°C et de manière particulièrement préférée de 200 à 230°C, de préférence sans addition d'un catalyseur.

15. Procédé selon la revendication 14, **caractérisé en ce que** l'on transforme comme composant de l'acide phosphorique une substance choisie parmi l'acide orthophosphorique, l'acide polyphosphorique et le décaoxyde de tétraphosphore, de préférence l'acide orthophosphorique.

16. Composition cosmétique, pharmaceutique ou dermatologique, **caractérisée en ce qu'**elle contient un ou plusieurs esters de l'acide phosphorique selon l'une ou plusieurs des revendications 1 à 13.

17. Composition selon la revendication 16, **caractérisée en ce qu'**elle se trouve sous forme d'une composition aqueuse, aqueuse-alcoolique ou aqueuse-tensioactive, d'une émulsion, d'une suspension, d'une dispersion, d'une poudre ou d'un spray.

18. Composition selon la revendication 16 ou 17, **caractérisée en ce qu'**elle présente un pH de 2 à 10, de préférence de 2 à 6, de manière particulièrement préférée de 2,5 à 5 et en particulier de préférence de 3 à 4,5.

19. Composition selon l'une ou plusieurs des revendications 16 à 18, **caractérisée en ce qu'**elle contient un ou plusieurs électrolytes.

20. Composition selon la revendication 19, **caractérisée en ce que** la teneur en ledit un ou lesdits plusieurs électrolyte(s), par rapport à la composition totale, est de 0,1 à 20,0% en poids, de préférence de 0,2 à 10,0% en poids et de manière particulièrement préférée de 0,5 à 5,0% en poids.

21. Composition selon l'une ou plusieurs des revendications 16 à 20, **caractérisée en ce qu'**elle contient du peroxyde d'hydrogène ou des substances libérant du peroxyde d'hydrogène et se trouve de préférence sous forme d'un gel ou d'une crème.

22. Composition selon l'une ou plusieurs des revendications 16 à 21, **caractérisée en ce qu'**elle contient une ou plusieurs substances tensioactives.

23. Composition selon l'une ou plusieurs des revendications 16 à 22, **caractérisée en ce qu'**elle contient ledit un ou lesdits plusieurs ester(s) de l'acide phosphorique selon l'une ou plusieurs des revendications 1 à 13, par rapport à la composition finie, en une quantité de 0,01 à 10,0% en poids, de préférence de 0,1 à 6,0% en poids et de manière particulièrement préférée de 0,5 à 3,0% en poids.

24. Utilisation d'un ou de plusieurs des esters de l'acide phosphorique selon l'une ou plusieurs des revendications 1 à 13 comme épaississant, agent conférant une consistance, émulsifiant, additif à effet sensoriel, agent de solubilisation, dispersant, lubrifiant, adhésif ou stabilisateur, de préférence comme épaississant.

25. Utilisation d'un ou de plusieurs esters de l'acide phosphorique selon l'une ou plusieurs des revendications 1 à 13 dans des formulations de déodorant ou d'antiperspirant, en particulier dans des formulations de déodorant ou d'antiperspirant contenant des sels d'aluminium, de préférence du chlorhydrate d'aluminium ou des sels complexes d'aluminium-zirconium, pour diminuer la formation de résidus blancs sur les vêtements après l'utilisation des formulations de déodorant ou d'antiperspirant sur la peau.
